(19) Europäisches Patentamt
European Patent Office
Office européen des brevets

(11) Numéro de publication : **0 389 370 B1**

# (12) FASCICULE DE BREVET EUROPEEN

(45) Date de publication du fascicule du brevet : **27.04.94 Bulletin 94/17**

(51) Int. Cl.$^5$ : **C07J 43/00,** C07J 1/00, A61K 31/56

(21) Numéro de dépôt : **90400784.6**

(22) Date de dépôt : **22.03.90**

(54) **Nouveaux stéroides 19-Nor 3-céto comportant une chaîne en 17 aminosubstituée, leur procédé de préparation et les intermédiaires de ce procédé,leur application comme médicaments et les compositions pharmaceutiques les contenant.**

(30) Priorité : **22.03.89 FR 8903742**

(43) Date de publication de la demande : **26.09.90 Bulletin 90/39**

(45) Mention de la délivrance du brevet : **27.04.94 Bulletin 94/17**

(84) Etats contractants désignés : **CH DE FR GB IT LI NL**

(56) Documents cités :
**WO-A-87/01706**
**WO-A-87/07895**
**GB-A- 1 169 073**

(73) Titulaire : **ROUSSEL-UCLAF**
**35, Boulevard des Invalides**
**F-75007 Paris (FR)**

(72) Inventeur : **Claussner, André**
**62 rue Marc Vieville**
**F-93250 Villemomble (FR)**
Inventeur : **Leclaire, Jacques**
**1, rue de l'Epine Montain**
**F-91300 Massy (FR)**
Inventeur : **Nedelec, Lucien**
**45, Boulevard de l'Ouest**
**F-93340 Le Raincy (FR)**
Inventeur : **Philibert, Daniel**
**16, rue Chevalier**
**F-94210 La Varenne Saint Hilaire (FR)**

(74) Mandataire : **Bourgouin, André et al**
**Département des Brevets ROUSSEL UCLAF**
**111, route de Noisy B.P. no 9**
**F-93230 Romainville (FR)**

EP 0 389 370 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouveaux stéroïdes 19-Nor 3-céto comportant une chaîne en 17 aminosubstituée, leur procédé de préparation et les intermédiaires de ce procédé, leur application comme médicaments et les compositions pharmaceutiques les contenant.

L'invention a pour objet les composés de formule (I) :

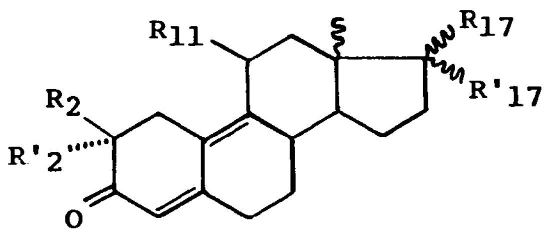

(I)

dans laquelle :

- $R_2$ et $R'_2$, identiques ou différents, représentent un atome d'hydrogène ou un radical méthyle,
- $R_{11}$ représente un radical organique renfermant de 1 à 18 atomes de carbone et éventuellement un ou plusieurs hétéroatomes, l'atome immédiatement adjacent à l'atome de carbone en 11 étant un atome de carbone,
- les traits ondulés en position 13 et 17 indiquent que lorsque le radical méthyle en position 13 est en alpha, le substituant $R_{17}$ est en configuration alpha et le substituant $R'_{17}$ est en configuration béta et que lorsque le radical méthyle en position 13 est en béta, le substituant $R_{17}$ est en configuration béta et le substituant $R'_{17}$ en configuration alpha et dans laquelle $R_{17}$ et $R'_{17}$ sont tels que

**soit a)** $R_{17}$ est un hydroxyle ou un radical acyloxy dérivé d'un acide organique carboxylique renfermant de 1 à 18 atomes de carbone et $R'_{17}$ est un radical

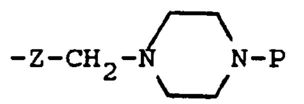

dans lequel Z est une simple liaison,
un radical alkylène renfermant de 1 à 5 atomes de carbone, un radical alcénylène ou un radical alcynylène renfermant de 2 à 5 atomes de carbone et P représente un radical pyrimidinyle ou un radical pyridyle, substitué par un ou deux radicaux identiques ou différents choisis parmi les radicaux amino, alkylamino, dialkylamino ou les hétérocycles aminés à 5 ou 6 chaînons, éventuellement substitués par un radical alkyle ayant de 1 à 3 atomes de carbone,

**soit b)** $R_{17}$ est un radical

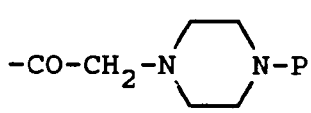

dans lequel P a la même signification que ci-dessus et $R'_{17}$ est un atome d'hydrogène un hydroxyle ou un radical acyloxy,
et les sels de ces composés.

$R_{11}$ peut représenter un radical alkyle saturé ou insaturé, linéaire ou ramifié ayant de 1 à 18 atomes de carbone.

Il s'agit alors de préférence du radical méthyle, éthyle, propyle, isopropyle, butyle, isobutyle ou tert-butyle, n-pentyle, n-hexyle, 2-méthyl pentyle, 2,3-diméthyl butyle, n-heptyle, 2-méthylhexyle, 2,2-diméthylpentyle, 3,3-diméthyl pentyle, 3-éthylpentyle, n-octyle, 2,2-diméthylhexyle, 3,3-diméthylhexyle, 3-méthyl-3-éthylpentyle, nonyle, 2,4-diméthyl heptyle, ou n-décyle. Il peut s'agir également des radicaux vinyle, isopropényle, allyle, 2-méthylallyle ou isobuténylе.

Les radicaux précités peuvent être substitués. Parmi les substituants possibles on peut citer les radicaux

thioalkyle tels que thiométhyle ou thioéthyle ; $R_{11}$ peut également être substitué par un ou plusieurs atomes d'halogène tels que fluor, chlore, brome, iode, ou par les radicaux amino substitués tels que diméthylamino.

$R_{11}$ peut également représenter un radical aryle tel que phényle ou aralkyle tel que benzyle. Ces radicaux aromatiques peuvent être substitués en ortho, méta ou para par un ou plusieurs radicaux alkyles renfermant de préférence de 1 à 4 atomes de carbone ; un ou plusieurs radicaux alkyloxy ayant préférentiellement de 1 à 4 atomes de carbone tels que les radicaux méthoxy, éthoxy, propyloxy, isopropyloxy, butyloxy, isobutyloxy, tert-butyloxy ; alkényloxy tel que vinyloxy ou allyloxy ; un ou plusieurs atomes d'halogène, de préférence chlore ou fluor ; un ou plusieurs radicaux choisis parmi les radicaux hydroxyle, trifluorométhyle, alkylthio ayant de 1 à 4 atomes de carbone éventuellement oxydé sous forme de sulfoxyde ou de sulfone tel que méthylthio, éthylthio ; ou un radical acyle tel que formyle, acétyle, propionyle, butyryle ou benzoyle, de préférence acétyle ; bien entendu les radicaux aryle ou aralkyle peuvent être substitués par une combinaison de ces différents radicaux tel que par exemple 3-fluoro, 4-diméthylamino phényle ;

$R_{11}$ peut également représenter un radical aryle hétérocyclique éventuellement substitué par les différents radicaux envisagés ci-dessus. On peut citer les radicaux thiényle, furyle, isothiényle, isofuryle, thiazolyle, isothiazolyle, oxazolyle, isoxazolyle, thiadiazolyle, pyridinyle ou pipéridinyle et les hétérocycles connus de l'homme de métier ;

$R_{11}$ peut également représenter un radical cycloalkyle tel que cyclopropyle, cyclobutyle, cyclopentyle ou cyclohexyle, cycloalkényle tel que cyclobutényle ou cyclopropényle ;

$R_{11}$ peut représenter également un radical comportant un noyau arylique substitué soit par une fonction amine éventuellement substituée par un ou deux radicaux alkyle ayant de 1 à 8 atomes de carbone, soit par un radical aminé incorporé dans un hétérocycle comportant éventuellement un autre hétéroatome choisi dans le groupe formé par l'oxygène, l'azote et le soufre, tel que les radicaux morpholino ou pipéridinyle.

Le noyau arylique est alors de préférence le noyau phényle.

Comme substituant sur le noyau arylique on peut également envisager un radical amino (substitué) alkyle, tel que le radical diméthylamino méthyle, diméthylamino éthyle ; un radical amino (substitué) alkyloxy tel que le radical diméthylamino éthyloxy.

On peut également citer les radicaux comportant un atome de silicium tel que le radical triméthylsilyl phényle.

Les radicaux précédemment cités comportant un atome d'azote peuvent être oxydés.

De manière générale, on préfère les produits dans lesquels le substituant $R_{11}$ comporte un hétéroatome de préférence l'azote ou le soufre.

**Selon a) :** lorsque $R_{17}$ est un radical acyloxy, il peut s'agir du dérivé d'un acide aliphatique ou cycloaliphatique saturé ou insaturé et notamment d'un acide alcanoïque tel que par exemple l'acide acétique, propionique, butyrique ou isobutyrique, valérique ou undécylique, d'un acide hydroxyalcanoïque tel que par exemple l'acide hydroxyacétique ; d'un acide cycloalkylcarboxylique ou (cycloalkyl) alcanoïque tel que par exemple l'acide cyclopropyl, cyclopentyl ou cyclohexylcarboxylique, cyclopentyl ou cyclohexyl acétique ou propionique, d'un acide benzoïque ou d'un acide phénylalcanoïque tel que l'acide phényl acétique ou phényl propionique, d'un amino acide tel que l'acide diéthylamino acétique ou aspartique ou de l'acide formique ; il s'agit de préférence du dérivé de l'acide acétique, propionique ou butyrique.

Dans $R'_{17}$, lorsque Z est un radical alkylène il s'agit de préférence d'un radical méthylène, éthylène ou triméthylène, lorsque Z est un radical alcénylène il s'agit de préférence d'un radical vinylène, lorsque Z est un radical alcynylène il s'agit de préférence d'un radical éthynylène et lorsque P est substitué par un radical alkylamino, le radical alkyle comporte de préférence de 1 à 4 atomes de carbone, il peut s'agir des radicaux méthyl, éthyl, n-propyl, isopropyl, n-butyl, isobutyl, tert-butylamino, de préférence du radical méthyl ou éthylamino, lorsque P est substitué par un radical dialkylamino, les radicaux alkyles comportent de préférence de 1 à 4 atomes de carbone, il peut s'agir des radicaux diméthyl, diéthyl ou méthyléthylamino, lorsque P est substitué par un ou deux hétérocycles aminés, il peut s'agir d'un hétérocycle saturé, de préférence une pyrrolidine ou une pipéridine, éventuellement substitué par un radical alkyle tel que méthyle, éthyle, propyle ou isopropyle, de préférence méthyle ou éthyle, ou d'un hétérocycle insaturé de préférence un pyrrole éventuellement substitué par un radical alkyle tel qu'un radical méthyle. Le ou les hétérocycles aminés sont en général liés à P par l'atome d'azote.

**Selon b) :** dans $R_{17}$, lorsque P est substitué par un radical alkylamino, dialkylamino ou par des hétérocycles aminés, il s'agit de l'un des radicaux ou de l'un des hétérocycles cités précédemment ;

et lorsque $R'_{17}$ est un radical acyloxy, il s'agit de préférence d'un dérivé choisi parmi ceux indiqués ci-dessus, particulièrement d'un dérivé de l'acide acétique, propionique, butyrique ou benzoïque.

L'invention s'étend naturellement aux sels des composés de formule (I), comme par exemple les sels formés avec les acides chlorhydrique, bromhydrique, nitrique, sulfurique, phosphorique, acétique, formique, propionique, maléique, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques, tels que l'acide benzoïque.

Parmi les composés de l'invention, on peut citer particulièrement les composés de formule (I) pour lesquels P représente un radical pyrimidinyle ou un radical pyridyle substitué par deux radicaux, identiques ou différents, choisis parmi les radicaux dialkylamino ou les hétérocycles aminés à 5 ou 6 chaînons.

Parmi les composés de l'invention, on peut citer tout particulièrement les composés de formule (I) pour lesquels P représente un radical

- 2,6-bis(1-pyrrolidinyl) 4-pyrimidinyle, un radical
- 5,6-bis(diéthylamino) 2-pyridyle ou un radical
- 3,6-bis(diéthylamino) 2-pyridyle

et ceux pour lesquels le radical méthyle en position 13 est en configuration béta.

Parmi les composés de l'invention, on peut citer notamment les composés de formule (I) pour lesquels $R_{11}$ représente un radical aryle ou aralkyle portant une fonction amine :

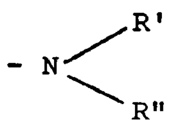

dans laquelle R' et R" représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote et le soufre, dont au moins un atome d'azote, ou substitué par un hétérocycle comportant au moins un atome d'azote, ceux pour lesquels $R_{11}$ représente un radical 2,3 ou 4-pyridyle,
un radical

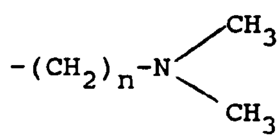

(n ≧ 3),
un radical :

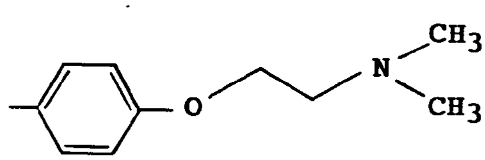 ,

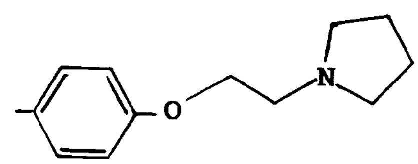 ,

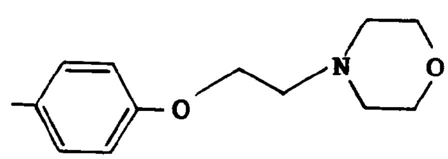 ,

un radical :

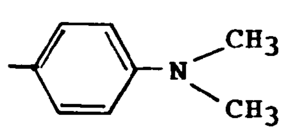 , 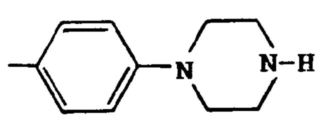 , 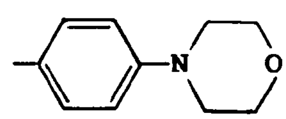

un radical :

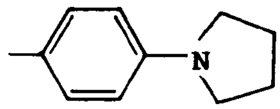 , 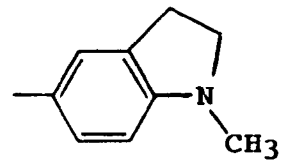

ou un radical :

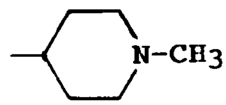

ceux pour lesquels $R_{11}$ représente un radical thiényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkyloxy, alkylthio éventuellement oxydé sous forme de sulfoxyde ou de sulfone et ceux pour lesquels $R_{11}$ représente un radical phényle substitué par un radical choisi dans le groupe formé par les radicaux chloro, fluoro, méthylthio, méthylsulfonyle, méthoxy, hydroxy, allyloxy et acyle tel qu'acétyle. Parmi ces derniers radicaux, on préfère le radical

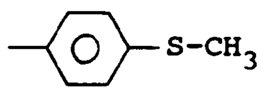 ou 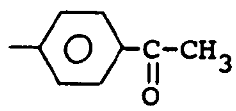

Parmi les composés préférés de l'invention on peut citer ceux pour lesquels $R_2$ et $R'_2$, identiques, représentent chacun un atome d'hydrogène, ceux pour lesquels $R_{17}$ est un hydroxyle lorsque $R'_{17}$ représente un radical

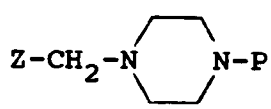

et plus particulièrement ceux pour lesquels Z est -C≡C- ou Z est -CH=CH- ou Z est $-CH_2-CH_2-$ lorsque $R'_{17}$ représente un radical

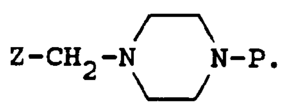

Parmi les composés préférés de l'invention on peut donc naturellement citer les composés dont la préparation est donnée plus loin dans la partie expérimentale à savoir :

- la 11béta-[4-(diméthylamino) phényl] 17alpha-[3-[4-[ 2,6-bis(1-pyrrolidinyl) 4-pyrimidinyl] 1-pipérazinyl] 1-propynyl] 17béta-hydroxy estra-4,9-dièn-3-one,
- la 11béta-[4-(diméthylamino) phényl] 17alpha-[3-[4-[5,6-bis(diéthylamino) 2-pyridyl] 1-pipérazinyl] 1-propynyl] 17béta-hydroxy estra-4,9-dièn-3-one ou
- la 11béta-[4-(diméthylamino) phényl] 17alpha-[3-[4-[3,6-bis(diéthylamino) 2-pyridyl] 1-pipérazinyl] 1-propynyl] 17béta-hydroxy estra-4,9-dièn-3-one ou
- la 11béta-[4-(diméthylamino) phényl] 17alpha-[3-[4-[2,6-bis(1-pyrrolidinyl) 4-pyrimidinyl] 1-pipérazinyl] 1-propényl] 17béta-hydroxy estra-4,9-dièn-3-one ou
- la 11béta-[4-(diméthylamino) phényl] 17alpha-[3-[4-[2,6-bis(1-pyrrolidinyl) 4-pyrimidinyl] 1-pipérazinyl] 1-propyl] 17béta-hydroxy estra-4,9-dièn-3-one et leurs sels.

L'invention a également pour objet un procédé de préparation des composés de formule (I) caractérisé en

ce que l'on soumet, dans un solvant neutre et en présence d'une base, un composé de formule (II) choisi parmi **soit a)** un composé de formule (IIa) :

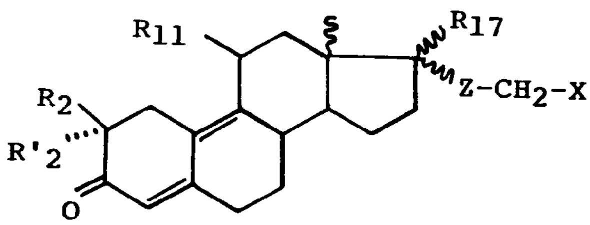

(IIa)

**soit b)** un composé de formule (IIb) :

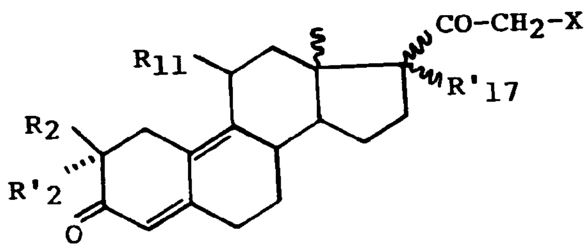

(IIb)

formules dans lesquelles X est un atome d'halogène et $R_2$, $R'_2$, $R_{11}$, les traits ondulés, $R_{17}$, $R'_{17}$ et Z ont les mêmes significations que précédemment, à l'action d'un composé de formule (III) :

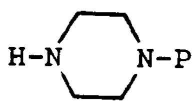

(III)

dans laquelle P a la même signification que précédemment et, le cas échéant, lorsque Z est un radical alcynylène ou alcénylène, l'on soumet les produits obtenus à une réaction d'hydrogénation partielle ou totale et, si désiré, l'on soumet les produits obtenus à l'action d'un acide pour obtenir le sel correspondant.

Les composés de formule (I) sont obtenus en utilisant un composé de formule (II) choisi parmi soit un composé de formule (IIa), soit un composé de formule (IIb), dans lequel X, peut être un atome de chlore, de brome ou d'iode, en opérant dans un solvant neutre tel que par exemple le diméthylformamide, le tétrahydrofuranne, le chlorure de méthylène, l'acétonitrile, l'éther éthylique ou l'acétone, en présence d'une base telle que par exemple un carbonate ou un bicarbonate de métal alcalin de préférence de sodium ou de potassium, la triéthylamine ou la diisopropyléthylamine.

Le cas échéant, on soumet les produits de formule (I) obtenus, renfermant un radical Z alcynylène ou alcénylène, à une réaction d'hydrogénation partielle ou totale, par exemple à l'aide d'hydrogène en présence de palladium sur charbon actif ou sur sulfate de baryum, en présence ou non de triéthylamine, afin d'obtenir des composés de formule (I) ayant un radical Z alcénylène ou alkylène.

L'invention a également pour objet une variante de préparation des composés de formule (I) dans laquelle $R_2$ et $R'_2$ représentent chacun un atome d'hydrogène, $R_{17}$ est un radical hydroxyle et $R'_{17}$ est un radical

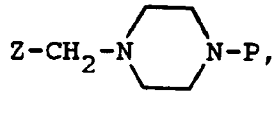

caractérisé en ce que l'on soumet un composé de formule (II') :

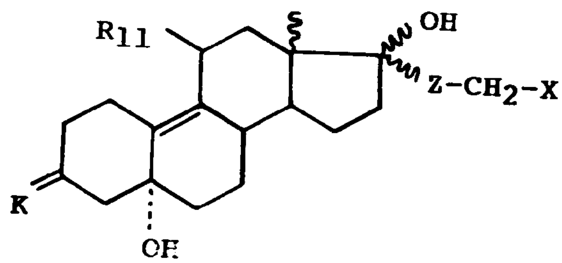

(II')

dans laquelle X est un atome d'halogène, $R_{11}$, les traits ondulés et Z ont la même signification que précédemment et K représente un groupement protecteur de la fonction cétone en 3, à l'action d'un composé de formule (III) :

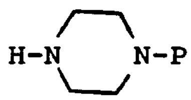

(III)

dans laquelle P a la même signification que précédemment puis soumet le produit de formule (XV) obtenu :

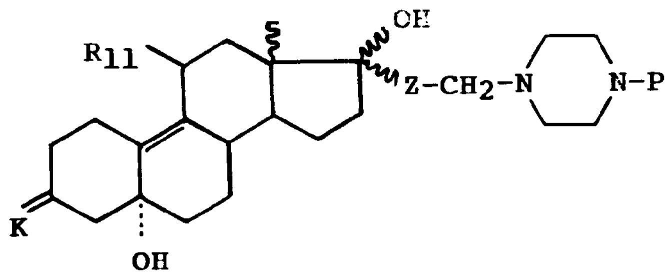

(XV)

à une réaction de deshydratation et d'hydrolyse susceptibles de libérer la fonction 3-céto delta-4, cette réaction étant précédée ou suivie d'une hydrogénation partielle ou totale éventuelle, lorsque Z est un radical alcynylène ou alcénylène, et si désiré, soumet les produits de formule (I) à l'action d'un acide pour obtenir le sel correspondant.

Selon la variante, les composés de formule (I) sont obtenus en faisant réagir un composé de formule (II') avec un composé de formule (III), dans les mêmes conditions opératoires que précédemment, puis en libérant la fonction 3-céto delta-4, par exemple par action d'acide chlorhydrique. Le cas échéant, on soumet le produit de formule (I) obtenu ou le produit de formule (XV) avant déblocage du groupement K à une réaction d'hydrogénation partielle ou totale effectuée dans les mêmes conditions que précédemment afin d'obtenir des composés de formule (I) ayant un radical Z alcénylène ou alkylène.

Dans un mode de réalisation préféré du procédé de l'invention et de la variante du procédé, le composé de formule (II) est le composé de formule (IIa) :

- 17alpha-(3-bromo 1-propynyl) 11béta-[4-(diméthylamino) phényl] 17béta-hydroxyestra-4,9-dièn-3-one et le composé de formule (II') est :
- le 3,3(éthanediyl bis oxy) 17alpha-(3-bromo 1-propynyl) 11béta-[4-(diméthylamino) phényl] 5alpha,17béta-dihydroxy estr-9-ène décrits ci-après, le solvant neutre est l'acétone, la base est le carbonate de potassium et le composé de formule (III) est soit :
- la 2,4-bis(1-pyrrolidinyl) 6-(1-pipérazinyl) pyrimidine :

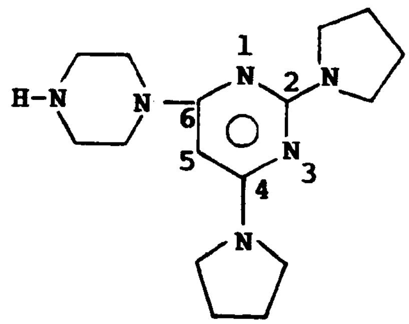

qui peut être obtenue selon la préparation décrite dans la demande de brevet WO 87/01706, soit :

- la N,N,N',N'-tétraéthyl 6-(1-pipérazinyl) 2,3-pyridine diamine

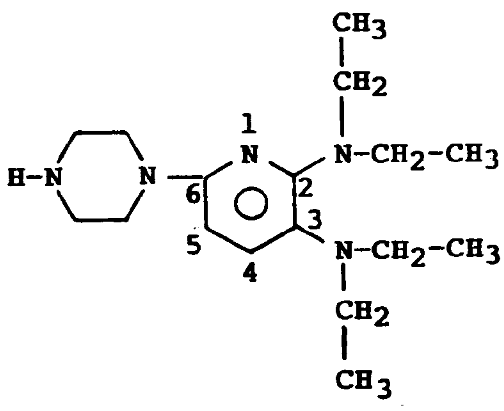

qui, bien que dénommée de façon erronée, peut être obtenue selon la préparation décrite dans la demande de brevet WO 87/01706 ou soit :

- la N,N,N',N'-tétraéthyl 6-(1-pipérazinyl) 2,5-pyridine diamine

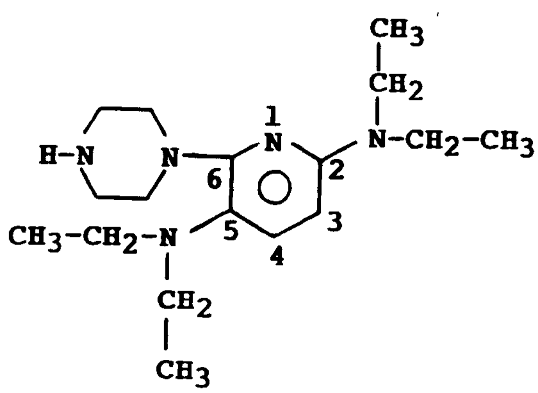

décrite dans la demande de brevet français n° 89-03740 ce même jour et dont la préparation est donnée plus loin dans la partie expérimentale et qui contrairement à ce qui est indiqué ne peut être obtenue selon la préparation décrite dans la demande de brevet WO 87/01706.

Les composés de formule (I) selon l'invention peuvent être obtenus à l'état de sels par des méthodes connues qui consistent à faire réagir le composé (I) avec un acide minéral ou organique choisi dans le groupe des acides cités ci-dessus.

Les composés de formule (I) ainsi que leurs sels présentent d'intéressantes propriétés pharmacologiques, notamment, une activité antioxydante par inhibition de la péroxydation lipidique tissulaire, par exemple au niveau du rein, du coeur, plus particulièrement au niveau du cerveau et de la moelle épinière. Les composés de formule (I) ainsi que leurs sels présentent aussi une activité détoxifiante dans les intoxications aigues associées à la péroxydation des lipides des tissus cérébraux tels que le cerveau ou la moelle épinière.

Les composés de formule (I) ainsi que leurs sels présentent en outre une activité anti-inflammatoire intéressante, par exemple dans les phénomènes de l'inflammation aiguë médiée par les dérivés de l'acide arachidonique.

Ces propriétés justifient leur application en thérapeutique.

L'invention a donc pour objet les composés de formule (I) à titre de médicaments et leurs sels avec les acides pharmaceutiquement acceptables.

Parmi les médicaments de l'invention, on peut citer tout particulièrement les composés des exemples 1 à 6 et leurs sels pharmaceutiquement acceptables.

Les médicaments selon l'invention peuvent être utilisés dans le traitement de désordres biologiques consécutifs à des traumatismes. On entend, par traumatismes, des dommages tissulaires dans lesquels interviennent la génération de péroxydes lipidiques et qui peuvent être provoqués par des agents variés, par exemple des agents physiques tels que des contusions, en particulier des contusions cérébrales associées ou non à des hémorragies locales, ou des agents chimiques tels que ceux utilisés en chimiothérapie antitumorale comme l'adriamycine ou tels que ceux utilisés en immunothérapie cancéreuse comme l'IL2 ou le TNF.

Ils sont surtout intéressants dans le traitement de l'ischémie cérébrale, en particulier dans le traitement de l'infarctus cérébral et dans la prévention de sa récidive, ou dans le traitement d'intoxications médicamenteuses provoquées par une chimiothérapie ou une immunothérapie ou l'association des deux.

Ils peuvent en outre être utilisés dans le traitement des réactions inflammatoires ; ils sont intéressants pour le traitement des réactions inflammatoires locales, comme par exemple les oedèmes, les dermatoses, les prurits, les diverses formes d'eczéma et les érythèmes solaires ou pour le traitement de maladies inflammatoires aiguës ou les maladies inflammatoires chroniques, par exemple la polyarthrite rhumatoïde, le psoriasis ou la sclérose en plaque.

Les médicaments de l'invention peuvent être administrés par voie orale ou par voie parentérale, telle qu'en injection intra-musculaire, intraarticulaire, intrathécale, de préférence en injection intraveineuse en bolus ou en perfusion continue ou par voie locale en application topique sur la peau ou les muqueuses.

L'invention s'étend aux compositions pharmaceutiques renfermant comme principe actif au moins l'un quelconque des médicaments ci-dessus.

Les compositions pharmaceutiques sont préparées selon les méthodes usuelles. Le principe actif peut être incorporé à des excipients habituellement employés dans ces compositions pharmaceutiques, tels que le talc, l'amidon, les véhicules aqueux ou non, les corps gras d'origine animale ou végétale, les dérivés paraffiniques, les glycols, les divers agents mouillants, dispersants ou émulsifiants et les conservateurs.

Les sels des produits de formule (I) , plus solubles dans l'eau, sont utilisés de préférence pour les formulations aqueuses destinées à l'injection intraveineuse.

La posologie utile, la fréquence et la durée du traitement qui dépendent de l'aminostéroïde et de la voie d'administration choisis varient, notamment en fonction du sujet et de la sévérité de l'affection à traiter. La dose peut être comprise entre 0,02 et 100 mg/kg/j, de préférence entre 0,01 et 1 mg/kg/j, par voie intraveineuse ou par voie intramusculaire et éventuellement répétée plusieurs fois par jour.

Les composés de formule (II) utilisés comme produits de départ sont des dérivés halogénés de stéroïdes 19-Nor 3-céto qui sont des produits nouveaux et l'invention a donc également pour objet les composés de formule (II) à titre de produits industriels nouveaux.

Les composés de formule (IIa) utilisés en tant que produits de départ du procédé de l'invention, sont préparés selon un mode opératoire, dont un exemple est donné plus loin dans la partie expérimentale.

De manière générale, les composés de formule (IIa) peuvent être préparés selon le schéma suivant :

Selon le procédé décrit dans la demande de brevet FR 2 433 536, on fait agir le lithien dérivé du produit de formule (IV) :

$$\text{H-Z-CH}_2\text{-OR} \qquad \text{(IV)}$$

dans laquelle Z a la signification donnée ci-dessus et R représente soit un atome d'hydrogène, soit un groupement protecteur de la fonction alcool, sur un stéroïde de formule (V) :

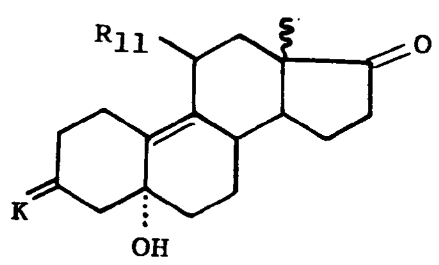

(V)

dans laquelle $R_{11}$ et le trait ondulé ont la signification indiquée ci-dessus et K est un groupement protecteur de la fonction cétone en 3 tel qu'un cétal cyclique, pour obtenir un produit de formule (VI) :

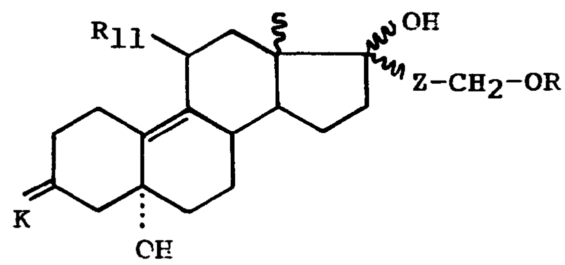

(VI)

que l'on soumet à un agent de deshydratation et d'hydrolyse susceptible de libérer la fonction 3-céto delta-4 et éventuellement la fonction alcool pour obtenir le produit de formule (VII) :

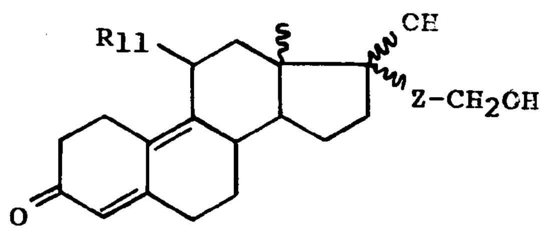

(VII)

que l'on soumet si désiré à un agent de méthylation, après avoir protégé provisoirement les fonctions alcool, pour obtenir le produit de formule (VIII) :

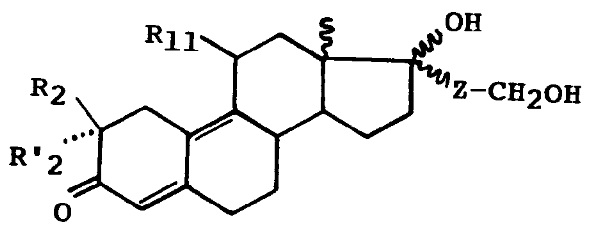

(VIII)

dans laquelle l'un au moin des substituants $R_2$ et $R_2$' représente un radical méthyle produits de formule (VII) ou (VIII) que l'on soumet à un agent d'halogénation pour obtenir le produit correspondant à un produit de formule (IIa) dans laquelle $R_{17}$ représente un radical hydroxyle.

Les produits obtenus peuvent être ensuite soumis, si désiré, à un agent d'estérification, par exemple selon le procédé décrit dans la demande de brevet FR 2 433 536, qui introduit le radical acyle en position 17 seulement.

Les produits de formule (V) telle que définie ci-dessus, nécessaires à la mise en oeuvre du procédé sont des stéroïdes 17 céto obtenus à partir des stéroïdes delta-9(11)5(10)époxy correspondants qui sont des produits connus, décrits par exemple dans le brevet EP 0057115.

Les stéroïdes de formule (V) ayant un radical méthyle en position 13 en configuration alpha sont préparés à partir des stéroïdes 17 céto en configuration 13 béta selon le procédé décrit dans le brevet EP 129499.

Les stéroïdes de formule (VIII) dans laquelle $R_2$ et $R'_2$ représentent un radical méthyle, sont préparés à partir des stéroïdes correspondants de formule (VII) selon le procédé décrit dans le brevet FR 2 528 434.

Les composés de formule (IIb) utilisés en tant que produits de départ du procédé de l'invention, sont des 19-nor, 3-céto-21 halostéroïdes qui peuvent être préparés, d'une manière générale, à partir des stéroïdes correspondants 21 hydroxylés selon des méthodes connues de l'homme de métier ou par exemple selon le procédé décrit dans la demande de brevet WO 87/1706. Les stéroïdes 19-nor, 3céto-21 hydroxylés ayant un atome d'hydrogène ou un hydroxyle en 17alpha sont des stéroïdes connus ou dont la préparation est connue de l'homme de métier tel que décrit par exemple dans les brevets FR 2 528 434 ou EP 129499. Ils peuvent aussi, par exemple, être préparés à partir des stéroïdes 17 céto correspondants en utilisant les procédés décrits

dans les demandes de brevets FR 2 462 445 ou FR 2 498 607, les stéroïdes 17 céto sont eux-mêmes des produits connus décrits par exemple dans les brevets FR 2 522 328 ou EP 104 387.

Les composés de formule (II') utilisés comme produits de départ sont des dérivés halogénés de stéroïdes 19-Nor 3-céto bloqués, qui sont des produits nouveaux et l'invention a donc également pour objet les composés de formule (II') à titre de produits industriels nouveaux.

Les composés de formule (II') utilisés en tant que produits de départ du procédé de l'invention sont préparés selon un mode opératoire dont un exemple est donné plus loin dans la partie expérimentale. De manière générale, ils sont obtenus à partir des produits de formule (VI) dans laquelle R est un atome d'hydrogène, que l'on soumet à un agent d'halogénation.

Parmi les produits industriels nouveaux de formule (II) et (II') préférés de l'invention, on peut citer les composés dont la préparation est donnée plus loin dans la partie expérimentale, à savoir :

- la 17alpha-(3-bromo 1-propynyl) 11béta-[4-(diméthylamino) phényl] 17béta-hydroxy estr-4,9-dièn-3-one et
- la 3,3-(éthanediyl bis oxy) 17alpha-(3-bromo 1-propynyl) 11béta-[4-(diméthylamino) phényl] 5alpha,17béta-dihydroxy estr-9-ène.

Les composés de formule (XV) sont des produits intermédiaires nouveaux et l'invention a donc également pour objet les composés de formule (XV) à titre de produits intermédiaires nouveaux.

Les composés de formule (III) utilisés comme produits de départ du procédé de l'invention, dans lesquels P est un radical non substitué ou monosubstitué, sont préparés selon la demande de brevet WO 87/1706 ainsi que les composés de formule (III) dans lesquels P est un radical disubstitué tel qu'un radical 2,6-(disubstitué) 4-pyrimidinyle, par exemple le radical :

- 2,6-bis(1-pyrrolidinyl) 4-pyrimidinyle

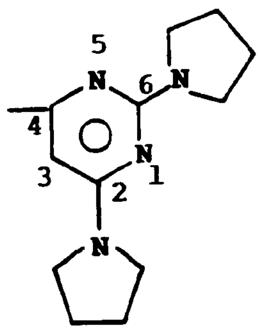

ou un radical :

- 5,6-(disubstitué) 2-pyridyle, par exemple le radical 5,6-bis(diéthylamino) 2-pyridyle

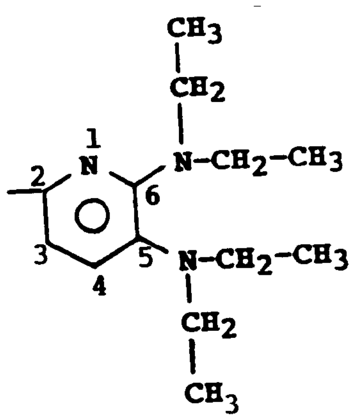

Par contre, les composés de formule (III) dans lesquels P est un radical 3,6-(disubstitué) 2-pyridyle, par exemple le radical 3,6-bis(diéthylamino) 2-pyridyle :

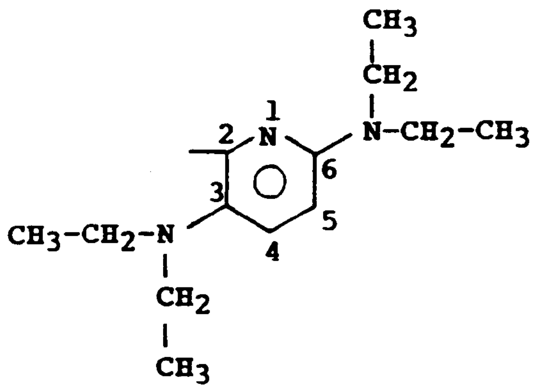

sont des produits nouveaux qui sont préparés tel que décrit dans la demande de brevet français n° 89-03740 selon un mode opératoire dont un exemple de préparation est donné plus loin dans la partie expérimentale.

Ces composés de formule (III) peuvent être préparés selon le schéma suivant : On fait agir un produit de formule (IX) :

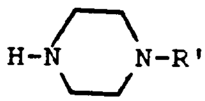

(IX)

dans laquelle R' représente un groupement protecteur de la fonction amino, par exemple un radical acétyle, sur un produit de formule (X) :

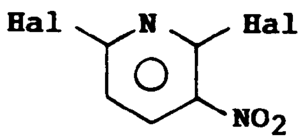

(X)

dans laquelle Hal représente un atome d'halogène, de préférence un atome de chlore, pour obtenir un produit de formule (XI) :

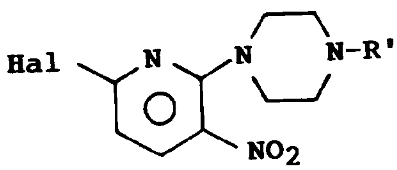

(XI)

sur lequel on fait réagir un produit de formule (XII) :

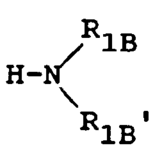

(XII)

dans laquelle $R_{1B}$ et $R_{1B}$' ont soit la signification indiquée ci-dessus pour $R_B$ et $R_B$' soit sont tels que ou bien l'un représente un groupement protecteur monovalent de la fonction amino, par exemple un groupement benzyle ou un groupement trityle, et l'autre représente un atome d'hydrogène ou bien $R_{1B}$ et $R_{1B}$' représentent ensemble un groupement protecteur divalent, par exemple $R_{1B}$ et $R_{1B}$' forment ensemble avec l'azote auquel ils sont liés un 2,5-diméthylpyrrole, pour obtenir un produit de formule (XIII) :

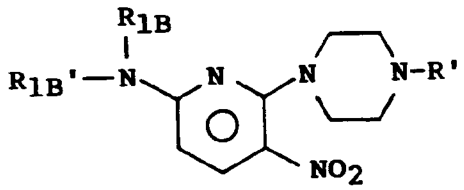

(XIII)

que l'on soumet à une réaction d'hydrogénation pour obtenir un produit de formule (XIV) :

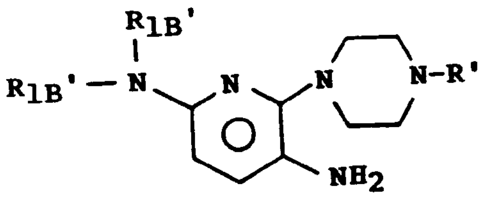

(XIV)

produit que si désiré :
**soit a)** l'on soumet à l'action d'un ou deux équivalents d'un derivé monohalogéné de $R_A$ ou de $R_A'$ pour obtenir un produit de formule (XIV') :

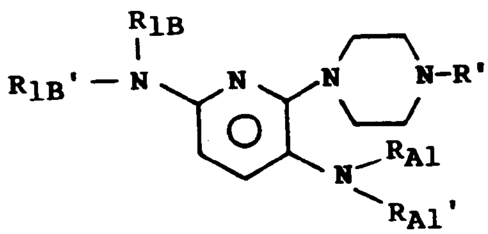

(XIV')

dans laquelle $R_{A1}$ et $R_{A1}'$ représentent soit l'un un atome d'hydrogène et l'autre un radical alkyle, soit tous les deux le même radical alkyle,
**soit b)** l'on soumet à l'action d'un dérivé monohalogéné de $R_A$ ou de $R_A'$, puis à l'action d'un dérivé monohalogéné de $R_A'$ ou de $R_A$ pour obtenir un produit de formule (XIV'') :

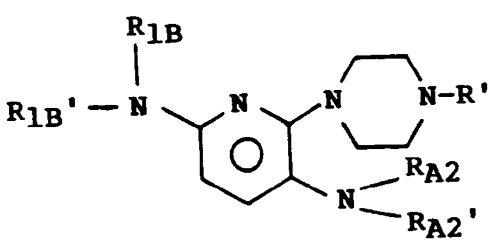

(XIV'')

dans laquelle $R_{A2}$ et $R_{A2}'$ représentent des radicaux alkyles différents,
**soit c)** l'on soumet à l'action d'un dérivé dihalogéné du butane ou du pentane, éventuellement substitué par un radical alkyle ayant de 1 à 3 atomes de carbone pour obtenir un produit de formule (XIV''') :

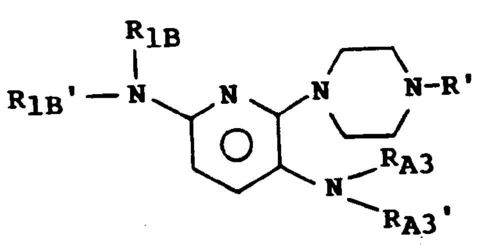

(XIV''')

dans laquelle $R_{A3}$ et $R_{A3}$' forment avec l'atome auquel ils sont liés un hétérocycle à 5 ou 6 chaînons, produits de formule (XIV), (XIV'), (XIV"), (XIV"') que l'on soumet à une réaction de déblocage du groupement R' et éventuellement des groupements $R_{1B}$ et/ou $R_{1B}$' pour obtenir les composés de formule (III) correspondants.

Parmi les produits nouveaux de formule (III) préférés de l'invention, on peut citer le composé dont la préparation est donnée plus loin dans la partie expérimentale, à savoir la N,N,N',N'-tétraéthyl 6-(1-pipérazinyl) 2,5 pyridine diamine qui, comme indiqué précédemment, ne peut être obtenue selon la préparation décrite dans la demande de brevet WO 87/01706. On peut également citer le composé de formule (III) à savoir la N,N'-dipyrrolidyl 6-(1-pipérazinyl) 2,5-pyridine diamine.

Les exemples suivants illustrent l'invention sans toutefois la limiter.

**Préparation 1 : 17alpha-(3-bromo 1-propynyl) 11béta-[4-(diméthylamino) phényl] 17béta-hydroxy estr-4,9-dièn-3-one.**

600 mg de 11 béta-[4-(diméthylamino) phényl] 17bétahydroxy 17alpha-(3-hydroxy 1-propynyl) estra-4,9-dièn-3-one décrit dans la demande de brevet FR 2 566 779 sont dissous dans 6 ml de chlorure de méthylène avec 491 mg de tétrabromure de carbone. La solution est refroidie à -10°C. On y ajoute alors goutte à goutte une solution de 531 mg de triphénylphosphine dans 3 ml de chlorure de méthylène. Le mélange est agité durant 20 minutes à -10°C. On le dispose alors tel quel sur une colonne de 15 g de silice et l'on élue avec le mélange Ether de pétrole (Eb 40 - 70°C)/Acétate d'éthyle 50/50. On recueille 384 mg de produit bromé pur sous forme de solide cristallisé.

**Préparation 2 : 3,3-(éthanediyl bis oxy) 11béta-(4-diméthylamino phényl) 17alpha-(3-bromo 1-propynyl) estr-9-ène 5alpha,17béta-diol.**

a) 3,3-(éthanediyl bis oxy) 11béta-(diméthylamino phényl) 17alpha-(3-hydroxy 1-propynyl) estr-9-ène.

On opère comme à la première partie de l'exemple 1 du brevet français FR 2 566 779 au départ d'alcool propargylique et de 3,3-(1,2-éthanediyl bis oxy) 11béta-(4-diméthylamino phényl) 5alpha-hydroxy estra-9-èn-17-one.

b) 3,3-(éthanediyl bis oxy) 11béta-(4-diméthylamino phényl) 17alpha-(3-bromo 1-propynyl) estra-9-ène 5alpha,17béta-diol.

On opère comme à la préparation 1 au départ de 4 g de produit obtenu au stade a). On obtient une huile que l'on utilise telle quelle à l'exemple 6.

**Préparation 3 : : N,N,N',N'-tétraéthyl 6-(1-pipérazinyl) 2,5-pyridinediamine.**

Stade A : 1-acétyl 4-[6-(diéthylamino) 3-nitro 2-pyridyl] pipérazine.

A un mélange de 78 g de 2,6-dichloro 3-nitro pyridine, 600 $cm^3$ d'acétonitrile et 66,3 g de carbonate de potassium, on ajoute en 50 minutes à 0°C, une solution de 51,22 g de N-acétyl pipérazine dans 200 $cm^3$ d'acétonitrile. On laisse revenir à température ambiante et agite 1 heure 15. On filtre les sels minéraux, ajoute au filtrat, 180 $cm^3$ de N-diéthylamine et 76 g de carbonate de potassium et chauffe le mélange 1 heure 15 au reflux, après refroidissement, on filtre les sels minéraux, et évapore à sec sous pression réduite, le résidu (166,9 g) est recristallisé dans 200 $cm^3$ d'acétate d'éthyle, on obtient 87,4 g de produit attendu. F = 120°C.

Stade B : N,N,N',N'-tétraéthyl 6-(1-pipérazinyl) 2,5-pyridinediamine.

On hydrogène sous une pression maximum de 1250 mbar à 25°C, un mélange de 70 g du produit obtenu au stade A, 1500 $cm^3$ de méthanol, 61,5 $cm^3$ d'acétaldéhyde et 10 g de charbon actif à 10 % de palladium. On absorbe environ 20 litres d'hydrogène, on filtre le catalyseur et évapore le filtrat à sec sous pression réduite. On reprend le résidu (89,8 g) avec 500 $cm^3$ de n-propanol et 91,3 g de potasse en pastilles, et chauffe 3 heures au reflux. On coule la solution refroidie dans 1 litre d'eau glacée et extrait avec du chlorure de méthylène, lave la solution organique avec une solution saturée de chlorure de sodium, sèche, filtre et concentre à sec, sous pression réduite. On chromatographie le résidu (58,9 g) sur silice (éluant : chlorure de méthylène-méthanolammoniaque (95-5-0,5)). On obtient 48,35 g de produit attendu que l'on utilise tel quel.

Analyse pour $C_{17}H_{31}N_5$

| | | | |
|---|---|---|---|
| Calculé : | C% 66,84 | H% 10,23 | N% 22,93 |
| Trouvé : | 66,9 | 10,5 | 22,6 |

**EXEMPLE 1 : 11béta-[4-(diméthylamino) phényl] 17alpha-[3-[4-[2,6-bis(i-pyrrolidinyl) 4-pyrimidinyl] 1-pipérazinyl] 1-propynyl] 17béta-hydroxy estra-4,9-dièn-3-one.**

A une solution de 7,98 g de 17alpha-(3-bromo 1 propynyl) 11béta-[4-(diméthylamino) phényl] 17béta-hydroxy estra-4,9-dièn-3-one (obtenu selon la préparation 1) dans 100 $cm^3$ d'acétone, on ajoute 4,77 g de carbonate de potassium puis 9,49 g de 2,4-bis(1-pyrrolidinyl) 6-(1-pipérazinyl) pyrimidine (préparé selon le brevet WO 87/01706) on agite 2 heures à température ambiante, dilue le milieu réactionnel avec de l'eau. On extrait avec du chlorure de méthylène et amène à sec sous pression réduite. On chromatographie l'extrait sec sur silice, (éluant : Acétate d'éthyle-cyclohexane (8-2)), on obtient 6,12 g de produit recherché que l'on chromatographie à nouveau sur silice (éluant : acétate d'éthyle-cyclohexane-isopropanol (80-20-4)), on obtient 5,5 g de produit recherché.
$[alpha]_D$ +103°5 ± 3° (c = 0,5 % EtoH)

Analyse pour $C_{45}H_{59}O_2N_7$

| | | | |
|---|---|---|---|
| Calculés : | C% 74,04 | H% 8,15 | N% 13,43 |
| Trouvés : | 74,1 | 8,2 | 13,2 |

Spectre IR ($CHCl_3$)

| | |
|---|---|
| Diènone | 1654 $cm^{-1}$ |
| | 1611 $cm^{-1}$ |
| 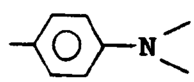 | 1518 $cm^{-1}$ |
| Absorption copule | 1562 $cm^{-1}$ |
| | 1355 $cm^{-1}$ |
| | 1453 $cm^{-1}$ |
| | 1445 $cm^{-1}$ |
| | 1437 $cm^{-1}$ |

**EXEMPLE 2 : Méthane sulfonate de 11béta-[4-(diméthylamino) phényl] 17alpha-[3-[4-[2,6-bis(1-pyrrolidinyl) 4-pyrimidinyl] 1-pipérazinyl] 1-propynyl] 17béta-hydroxy estra-4,9-dièn-3-one.**

A une solution de 2,57 g de produit obtenu comme à l'exemple 1 dans 30 $cm^3$ d'acétate d'éthyle, on ajoute lentement 11,6 $cm^3$ d'une solution 0,3 M d'acide méthane sulfonique dans l'acétate d'éthyle. On évapore le solvant sous pression réduite, reprend le résidu avec 2 $cm^3$ d'éthanol et ajoute à la solution obtenue 10 $cm^3$ d'éther isopropylique. On agite 30 minutes et essore le produit attendu (2,74 g). On recristallise 2,425 g de celui-ci par dissolution dans 7,5 $cm^3$ de chlorure de méthylène et précipitation par addition lente de 40 $cm^3$ d'éther isopropylique. On obtient 2,14 g de produit recherché. F -~ 194°C.

Analyse pour $C_{46}H_{63}N_7SO_5$

| | | | | |
|---|---|---|---|---|
| Calculés : | C% 66,88 | H% 7,69 | N% 11,87 | S% 3,88 |
| Trouvés : | 66,5 | 7,6 | 11,7 | 4,8 |

Spectre IR ($CHCl_3$)

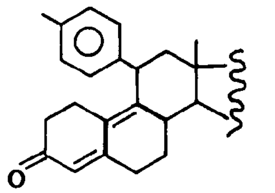

1654 $cm^{-1}$
1611 $cm^{-1}$
1518 $cm^{-1}$

**EXEMPLE 3 : 11béta-[4-(diméthylamino) phényl] 17alpha-[3-[4-[5,6-bis(diéthylamino) 2-pyridyl] 1-pipérazinyl] 1-propynyl] 17béta-hydroxy estra-4,9-dièn-3-one.**

On agite pendant 1 heure à température ambiante, 2,16 g de 17alpha-(3-bromo 1-propynyl) 11béta-[4-(diméthylamino) phényl] 17béta-hydroxy-estr-4,9-dièn-3-one (obtenu selon la préparation 1), 40 $cm^3$ d'acétone 2,7 g de N,N,N',N'-tétraéthyl 6-(1-pipérazinyl) 2,3-pyridinediamine (préparée selon le brevet WO 87/01706) et 1,27 g de carbonate de potassium. On filtre, concentre le filtrat à sec sous pression réduite et chromatographie le résidu sur silice (éluant : acétate d'éthyle-cyclohexane (8-2)) on obtient 2,9 g de produit recherché.
$[alpha]_D$ +93° ± 3° (c = 0,5 % EtoH)

Analyse pour $C_{46}H_{64}N_6O_2$

| | | | |
|---|---|---|---|
| Calculés : | C% 75,37 | H% 8,80 | N% 11,46 |
| Trouvés : | 75,6 | 8,9 | 12,2 |

Spectre IR ($CHCl_3$)

| | |
|---|---|
| OH | 3600 $cm^{-1}$ |
| Diènone | 1654 $cm^{-1}$<br>1612 $cm^{-1}$<br>865 $cm^{-1}$ |
| Système conjugué dû au réactif | 1587 $cm^{-1}$<br>1565 $cm^{-1}$<br>1478 $cm^{-1}$ |
| Aromatique | 1518 $cm^{-1}$ (F) type Ø - N< |

**EXEMPLE 4 : 17alpha-[3-[4-[3,6-bis(diéthylamino) 2-pyridyl] 1-pipérazinyl] 1-propynyl] 11béta-[4-(diméthylamino) phényl] 17béta-hydroxy estra-4,9-dièn-3-one.**

On opère comme à l'exemple 1 à partir de 3,42 g de 17alpha-(3-bromo 1 propynyl) 11béta-[4-(diméthylamino) phényl] 17béta-hydroxy estra-4,9-dièn-3-one (obtenu selon la préparation 1) et 4,29 g N,N,N',N'-tétraéthyl-6-(1-pipérazinyl) 2,5-pyridinediamine préparée selon la demande de brevet n° 89 03740 et obtenue selon le stade B de la préparation 3. On obtient 2,18 g de produit recherché.
$[alpha]_D$ +59° ± 2°5 (c = 0,5 % EtoH)

Analyse pour $C_{46}H_{64}N_6O_2$

| | | | |
|---|---|---|---|
| Calculés : | C% 75,37 | H% 8,80 | N% 11,46 |
| Trouvés : | 75,5 | 8,9 | 11,6 |

Spectre IR ($CHCl_3$)

| | | |
|---|---|---|
| OH | 3600 $cm^{-1}$ | |
| Diènone | 1654 $cm^{-1}$ | |
| | 1611 $cm^{-1}$ | |
| 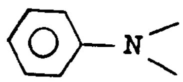 | 1518 $cm^{-1}$ | |
| | 1595 $cm^{-1}$ | Copule |
| | 1561 $cm^{-1}$ | |
| | 1486 $cm^{-1}$ | |

**EXEMPLE 5 : 17alpha-[3-[4-[2,6-bis(1-pyrrolidinyl) 4-pyrimidinyl] 1-pipérazinyl] 1-propényl] 11béta-[4-(diméthylamino) phényl] 17béta-hydroxy estra-4,9-dièn-3-one.**

On soumet pendant 4 heures à une hydrogénation sous une pression de 1261 mm de mercure un mélange de 2,2 g de produit obtenu à l'exemple 2, 140 $cm^3$ d'éthanol, 3 $cm^3$ de triéthylamine et 0,2 g de palladium à 10 % sur sulfate de baryum. On filtre le catalyseur, lave et distille les solvants sous pression réduite. On obtient 2,5 g de mousse jaune. Après purification par chromatographie sur silice (éluant : acétate d'éthyle-cyclohexane (8-2) et 2 % d'isopropanol) , on obtient le produit attendu.

Spectre IR ($CHCl_3$)

| | |
|---|---|
| Cétone conjuguée | 1654 $cm^{-1}$ |
| hétéroaromatique | 1612 $cm^{-1}$ |
| aromatique | 1563 $cm^{-1}$ |
| | 1555 $cm^{-1}$ |
| | 1518 $cm^{-1}$ |

Spectre UV

1) Dans EtoH
   Max 233 nm $E_1$' = 726 Epsilon = 53100
   Max 288 nm $E_1$' = 387 Epsilon = 28300
   Inf. 260, 305, 330.
2) Dans EtoH, HCl 0,1 N
   Max 244 nm $E_1$' = 446 Epsilon = 32600
   Max 300 nm $E_1$' = 543 Epsilon = 39700
   Inf. 218 nm.

**EXEMPLE 6 : 17alpha-[3-[4-[2,6-bis(1-pyrrolidinyl) 4-pyrimidinyl] 1-pipérazinyl] 1-propyl] 11béta-[4-(diméthylamino) phényl] 17béta-hydroxy estra-4,9-dièn-3-one.**

Stade A : 17alpha-[3-[4-[2,6-bis(1-pyrrolidinyl) 4-pyrimidinyl] 1-pipérazinyl] 1-propynyl] 11béta-[4-(diméthylamino) phényl] 3,3-(éthanediyl bis oxy) estra-9-ène 5alpha,17béta-diol.

On fait agir, dans les conditions indiquées à l'exemple 1, 3,54 g de 2,4-bis(1-pyrrolidinyl) 6-(1-piperazinyl) pyrimidine sur le 3,3-(éthanediyl bis oxy) 11béta-(4-diméthylamino phényl) 17alpha-(3-bromo 1-propynyl) estra-9-ène 5alpha,17béta-diol obtenu selon la préparation 2 à partir de 4 g de produit 17alpha-(3-hydroxy 1-propynyl) correspondant et obtient 5,26 g de produit attendu.

Spectre IR ($CHCl_3$)

| | | |
|---|---|---|
| Aromatique | 1614 $cm^{-1}$ | |
| 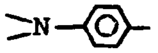 | 1518 $cm^{-1}$ | |
| Systèmes conjugués | 1566 $cm^{-1}$ | (maximum) |
| | 1544 $cm^{-1}$ | (épaulement) |

Stade B : 17alpha-[3-[4-[2,6-bis(1-pyrrolidinyl) 4-pyrimidinyl] 1-pipérazinyl] 1-propyl] 11béta-[4-(diméthylamino) phényl] 3,3-(éthanediyl bis oxy) estra-9-ène 5alpha,17béta-diol.

On soumet 3 heures à une hydrogénation sous 1250 mm de mercure, un mélange de 5,26 g de produit obtenu au stade A, 250 $cm^3$ d'éthanol, 2 g de palladium à 10 % sur sulfate de baryum, puis ajoute 2 g de catalyseur et poursuit 2 heures l'hydrogénation. Après filtrage et purification, on obtient 5,17 g de produit attendu.

Spectre IR ($CHCl_3$)

| | |
|---|---|
| OH | 3616 $cm^{-1}$ |
| Aromatique | 1564 $cm^{-1}$ |
| | 1554 $cm^{-1}$ |
| | 1518 $cm^{-1}$ |
| | 1468 $cm^{-1}$ |

Stade C : 17alpha-[3-[4-[2,6-bis(1-pyrrolidinyl) 4-pyrimidinyl] 1-pipérazinyl] 1-propyl] 11béta-[4-(diméthylamino) phényl] 17béta-hydroxy estra-4,9-dièn-3-one.

On agite à température ambiante 5,17 g de produit obtenu au stade B, dans 100 $cm^3$ d'éthanol et 100 $cm^3$ d'acide chlorhydrique. Après neutralisation, séchage et purification puis filtrage sur silice dans les conditions usuelles, on obtient 1,94 g de produit attendu.

$[alpha]_D$ +108° ± 3° (c = 0,45 % dans EtOH)

Spectre IR ($CHCl_3$)

| | |
|---|---|
| Diènone | 1654 $cm^{-1}$ |
| Système conjugué | 1611 $cm^{-1}$ |
| aromatiques | 1581 $cm^{-1}$ |
| hétérocycles | 1545 $cm^{-1}$ |
| | 1518 $cm^{-1}$ |

**ETUDE PHARMACOLOGIQUE**

I - Activité antioxydante.

L'activité antioxydante est recherchée, in vitro, par le test de formation de la malonyldialdéhyde (MDA) qui mesure la péroxydation lipidique déclenchée

**soit a)** de façon non enzymatique par le sulfate ferreux, dans

1) des homogénats de cerveau ou

2) des microsomes hépatiques de rat

**soit b)** de façon enzymatique par NADPH et le tétrachlorure de carbone, dans des microsomes hépatiques de rat.

**1.1** - La formation de MDA est mesurée sur des homogénats au 1/10° (V/V) de cerveaux de rat S.D (200 g) préparés dans un tampon de Krebs pH 7.4 selon les conditions décrites dans J. Biol. Chem. 262 (1987) 10438-10440. On incube 1 ml d'homogénat pendant 60 mn à 37°C en présence de 25 microlitres d'éthanol ou d'eau ou d'un mélange des deux, selon le produit, contenant ou non le produit à tester, après addition de 25 microlitres de solution de sulfate ferreux préparée extemporanément dans de l'eau dégazée à l'argon (200 microlitres final). On prélève 0,25 ml de mélange incubé et ajoute 1,5 ml d'acide phosphorique à 1 %, 0,25 ml de solution 200 microlitres de déféroxamine (Desféral R Ciba Geigy) dans l'eau, 10 microlitres de tert-butylhydroxytoluène

(BHT) à 8,7 mg/ml dans l'éthanol et 0,5 ml d'acide thiobarbiturique (TBA) à 0,6 % dans l'eau. On chauffe le mélange à 100°C pendant 45 mn, refroidit, ajoute 4 ml de n-butanol, centrifuge pendant 15 mn à 4000 t/mn puis lit la DO à 535 nm de la fraction surnageant. On incube dans les mêmes conditions les blancs de réaction en absence de Fe++. On calcule le pourcentage d'inhibition :

$$\text{pourcentage d'inhibition} = \frac{\text{DO en présence de produit}}{\text{DO en absence de produit}}$$

Résultats :

| concentration | pourcentage d'inhibition 5.10$^{-4}$M | 1.10$^{-4}$M | 1.10$^{-5}$M |
|---|---|---|---|
| produit testé | | | |
| produit obtenu à l'exemple 1 | 47,5 ± 8,8 | 42,6 ± 7,5 | 9,9 ± 6,4 |
| produit obtenu à l'exemple 2 | 60,9 ± 3,3 | 59,6 ± 4,4 | 16,0 ± 5,0 |
| produit obtenu à l'exemple 3 | 59,1 ± 3,2 | 49,2 ± 2,3 | 32,6 ± 10,1 |
| produit obtenu à l'exemple 4 | 71,8 ± 10,2 | 58,9 ± 10,9 | 48,6 ± 6,2 |

**1.2** - La formation de MDA est mesurée sur des microsomes hépatiques de rat S.D (200 g) préparés à partir de la fraction sédimentée à 100.000 G d'un homogénat de foie dans un tampon sucrose dont on utilise la fraction restant insoluble à 100.000 G dans un tampon de pyrophosphate de sodium 100 mM pH 7.4 et que l'on homogénéise dans un tampon phosphate de sodium 100 mM pH 7.4 à 20 % de glycérol, puis que l'on conserve à -80°C.

On incube les microsomes pendant 15 mn à 37°C dans 1 ml contenant du tampon Tris, HCl 35 mM, KCl 0,1 M pH 7.4, 1 mg de protéine microsomale, 5 microlitres d'éthanol renfermant ou non le produit à tester, 250 microlitres de solution d'ascorbate dans le tampon Tris (0,5 mM final), après l'addition de 250 microlitres de sulfate ferreux préparée extemporanément dans le tampon Tris (6 microlitres final). On arrête la réaction par addition de 2 ml d'une solution d'acide trichloracétique 1 M dans l'acide chlorhydrique 0,25 M contenant 0,4 % d'acide thiobarbiturique. On chauffe le mélange à 85°C pendant 25 mn, refroidit, centrifuge pendant 15 mn à 3500 t/mn puis lit la DO à 535 nm de la fraction surnageante. On effectue en même temps les blancs de réaction en absence de Fe++. On calcule le pourcentage d'inhibition comme précédemment.

Résultats :

| concentration | pourcentage d'inhibition 1.10$^{-5}$M | 5.10$^{-6}$M | 1.10$^{-6}$M | 1.10$^{-7}$M |
|---|---|---|---|---|
| produit testé | | | | |
| produit obtenu à l'exemple 1 | 76 ± 0,8 | 47 | 4 ± 0,6 | |
| produit obtenu à l'exemple 2 | 98,5 | 91 | 48 ± 1 | |
| produit obtenu à l'exemple 3 | 99,9 | 99,5 | 78,2 ± 0,9 | 5,2 ± 1 |
| produit obtenu à l'exemple 4 | 99,5 | 99,4 | 70 ± 2 | 0 ± 2 |

2 - La formation de MDA est mesurée sur des microsomes hépatiques de rats, prétraités au phénobarbital (80 mg/kg en 3 injections i.p), préparés comme décrit précédemment. On incube les microsomes pendant 15 mn à 37°C dans l ml contenant du tampon phosphate 0,1 M pH 7.4, 1 mg de protéine microsomale, 5 microlitres d'éthanol renfermant ou non le produit à tester, 5 microlitres de tétrachlorure de carbone (5,5 mM final) après l'addition de 50 microlitres de solution de NADPH dans le tampon phosphate (1 mM final). On arrête la réaction puis effectue le dosage selon les conditions décrites précédemment.

| Résultats : | pourcentage d'inhibition | | |
|---|---|---|---|
| concentration | $1.10^{-5}$M | $5.10^{-6}$M | $1.10^{-6}$M |
| produit testé | | | |
| produit obtenu à l'exemple 1 | 72 ± 3 | 58 ± 5 | 14 ± 4 |
| produit obtenu à l'exemple 2 | 91 ± 3 | 82 ± 3 | 28 ± 3 |
| produit obtenu à l'exemple 3 | 97 | 88 ± 2 | 40 ± 1 |
| produit obtenu à l'exemple 4 | 888 ± 1 | 85 ± 2 | 48 ± 3 |

II - Activité détoxifiante in vivo.

L'activité détoxifiante après intoxication associée à une péroxydation lipidique a été recherchée par mesure de la mortalité induite chez le rat par intoxication au nitrilotriacétate ferrique (NTA-Fe+++).

On prépare extemporanément une solution de complexe de NTA-Fe+++ que l'on met en suspension dans l'huile de sésame et que l'on injecte par voie i.p à des doses de 5,10 ou 15 mg de Fer/Kg de poids corporel à des rats mâles Wistar, non à jeun, d'environ 200 g.

On détermine le taux de survie des rats ayant reçu une injection préalable de produit à tester à la dose de 5 mg/Kg, par voie i.p, 30 mn avant l'injection de 15 mg/Kg de toxique.

Alors qu'aucun des animaux traités par le toxique seul n'a survécu après 4 heures, 20 % des animaux prétraités par le produit obtenu à l'exemple 2 étaient encore vivants après 15 jours.

III - Activité anti-inflammatoire.

L'activité anti-inflammatoire est évaluée in vivo par mesure de l'activité anti-oedémateuse à l'aide du test de l'oedème plantaire provoqué par l'acide arachidonique décrit par M.J. Di Martino et al. (Agents and Actions 1987 21 3/4 303).

Les animaux d'expérimentation sont des rats mâles EOPS de souche Sprague Dawley pesant 150-170 g (Iffa Credo).

Ce test est pratiqué sur des groupes de 8 rats mâles pesant 130 à 150 g à jeun depuis 16 heures. L'acide arachidonique est injecté sous l'aponévrose plantaire d'une patte postérieure à la dose de 0,2 mg sous un volume de 0,1 ml. Le volume de la patte est mesuré à l'aide d'un pléthysmomètre à eau, avant et 1 heure après l'injection de l'acide arachidonique. La différence entre ces deux volumes représente le degré d'inflammation. Les animaux sont traités par les produits à étudier ou le véhicule seul en même temps que l'injection de l'acide arachidonique.

Les produits étudiés ont été administrés par voie orale sous un volume de 4 ml/kg après avoir été mis en suspension dans la méthylcellulose à 0,5%. Toutes les expériences ont été réalisées avec comme produit de référence la dexaméthasone à la dose de 0,5 mg/kg par voie orale.

Les résultats sont exprimés en variation de volume de la patte 1 heure après l'injection d'acide arachidonique (AA), en absence ou en présence du produit à tester administré à différentes doses.

L'interprétation statistique des résultats a été effectuée selon le test de Dunnett (* $p < 0,05$ - ** $p < 0,01$) ou selon le test de Mann Whitney (° $p < 0,05$ - °° $p < 0,01$).

Pour chaque dose administrée par voie orale, on calcule le pourcentage d'inhibition de l'oedème par le produit testé, par rapport au témoin.

| | Dose mg/kg | Variation de volume de la patte 1 heure après AA(cm3) (moyenne ± e.s.m.) | % inhibition |
|---|---|---|---|
| Témoins | 0 | 0,58 ± 0,03 | |
| Produit de l'exemple 3 | 1 | 0,48 ± 0,05** | - 17 |
| | 4 | 0,43 ± 0,06** | - 27 |
| | 20 | 0,40 ± 0,04** | - 31 |

Le produit de l'exemple 3 manifeste une activité anti-inflammatoire significative à faible dose.

Dans les mêmes conditions expérimentales, la dexaméthasone induit une inhibition de l'oedème de 30 à 40% à la dose de 0,5 mg/kg.

**Revendications**

**1.** Les composés de formule (I) :

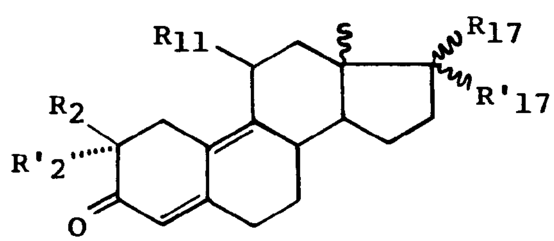

(I)

dans laquelle :

- $R_2$ et $R'_2$, identiques ou différents, représentent un atome d'hydrogène ou un radical méthyle,
- $R_{11}$ représente un radical organique renfermant de 1 à 18 atomes de carbone et éventuellement un ou plusieurs hétéroatomes, l'atome immédiatement adjacent à l'atome de carbone en 11 étant un atome de carbone,
- les traits ondulés en position 13 et 17 indiquent que lorsque le radical méthyle en position 13 est en alpha, le substituant $R_{17}$ est en configuration alpha et le substituant $R'_{17}$ est en configuration béta et que lorsque le radical méthyle en position 13 est en béta, le substituant $R_{17}$ est en configuration béta et le substituant $R'_{17}$ est en configuration alpha et dans laquelle $R_{17}$ et $R'_{17}$ sont tels que

**soit a)** $R_{17}$ est un hydroxyle ou un radical acyloxy dérivé d'un acide organique carboxylique renfermant de l à 18 atomes de carbone et $R'_{17}$ est un radical

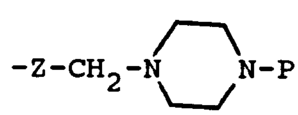

dans lequel Z est une simple liaison, un radical alkylène renfermant de 1 à 5 atomes de carbone, un radical

alcénylène ou un radical alcynylène renfermant de 2 à 5 atomes de carbone et P représente un radical pyrimidinyle ou un radical pyridyle, substitué par un ou deux radicaux identiques ou différents choisis parmi les radicaux amino, alkylamino, dialkylamino ou les hétérocycles aminés à 5 ou 6 chaînons, éventuellement substitués par un radical alkyle ayant de 1 à 3 atomes de carbone,

**soit b)** $R_{17}$ est un radical

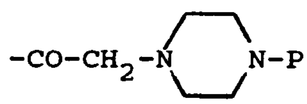

dans lequel P a la même signification que ci-dessus et $R'_{17}$ est un atome d'hydrogène un hydroxyle ou un radical acyloxy,

et les sels de ces composés.

**2.** Les composés de formule (I) telle que définie à la revendication 1 pour lesquels P représente un radical pyrimidinyle ou un radical pyridyle substitué par deux radicaux, identiques ou différents, choisis parmi les radicaux dialkylamino ou les hétérocycles aminés à 5 ou 6 chaînons.

**3.** Les composés de formule (I) telle que définie à la revendication 1 ou 2 pour lesquels P représente un radical

- 2,6-bis(1-pyrrolidinyl) 4-pyrimidinyle, un radical
- 5,6-bis(diéthylamino) 2-pyridyle ou un radical
- 3,6-bis(diéthylamino) 2-pyridyle.

**4.** Les composés de formule (I) telle que définie à l'une quelconque des revendications 1 à 3 pour lesquels le radical méthyle en position 13 est en configuration béta.

**5.** Les composés de formule (I) telle que définie à l'une quelconque des revendications 1 à 4 pour lesquels $R_{11}$ représente un radical aryle ou aralkyle portant une fonction amine :

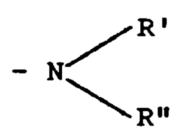

dans laquelle R' et R" représentent un radical alkyle renfermant de 1 à 8 atomes de carbone ou un radical alkyle primaire, secondaire ou tertiaire renfermant de 1 à 8 atomes de carbone, comportant un ou plusieurs hétéroatomes choisi dans le groupe constitué par l'oxygène, l'azote et le soufre, dont au moins un atome d'azote, ou substitué par un hétérocycle comportant au moins un atome d'azote.

**6.** Les composés de formule (I) telle que définie à l'une quelconque des revendications 1 à 4 pour lesquels $R_{11}$ représente un radical 2,3 ou 4-pyridyle,

un radical

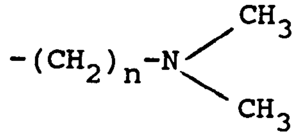

($n \geqq 3$),

un radical :

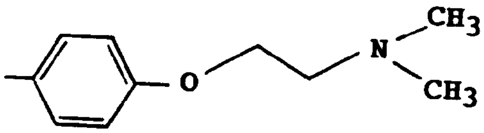

,

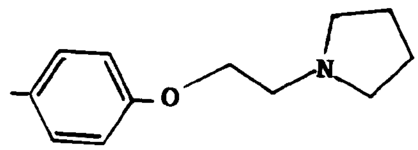

,

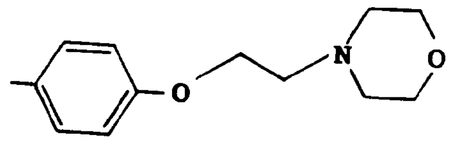

,

un radical :

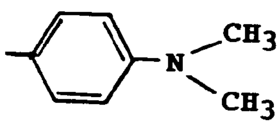

,

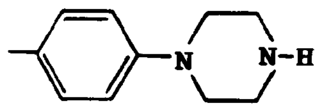

,

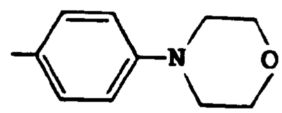

un radical :

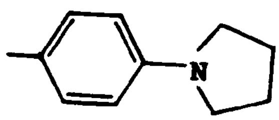

,

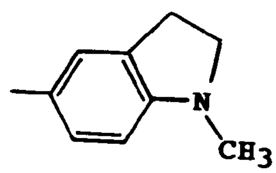

ou un radical :

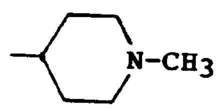

**7.** Les composés de formule (I) telle que définie à l'une quelconque des revendications 1 à 4 pour lesquels $R_{11}$ représente un radical thiényle, furyle, cycloalkyle ayant de 3 à 6 atomes de carbone ou phényle éventuellement substitué par un ou plusieurs radicaux choisis parmi les radicaux hydroxy, halogène, trifluorométhyle, alkyle, alkyloxy, alkylthio éventuellement oxydé sous forme de sulfoxyde ou de sulfone.

**8.** Les composés de formule (I) telle que définie dans les revendications 1 à 4 et 7 pour lesquels $R_{11}$ représente un radical phényle substitué par un radical choisi dans le groupe formé par les radicaux chloro, fluoro, méthylthio, méthylsulfonyle, méthoxy, hydroxy, allyloxy et acyle tel qu'acétyle.

**9.** Les composés de formule (I) telle que définie à l'une quelconque des revendications 1 à 8 pour lesquels $R_2$ et $R'_2$, identiques, représentent chacun un atome d'hydrogène.

**10.** Les composés de formule (I) telle que définie à l'une quelconque des revendications 1 à 9 pour lesquels $R_{17}$ est un hydroxyle lorsque $R'_{17}$ représente un radical

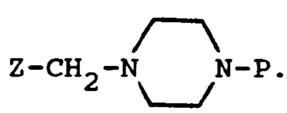

**11.** Les composés de formule (I) telle que définie à l'une quelconque des revendications 1 à 10 pour lesquels Z = -C≡C- ou Z = -CH=CH- ou Z= $-CH_2-CH_2-$ lorsque $R'_{17}$ représente un radical

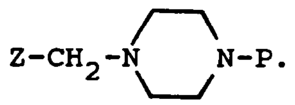

**12.** Les composés répondant à l'une quelconque des formules :

- 11béta-[4-(diméthylamino) phényl] 17alpha-[3-[4-[(2,6-bis(1-pyrrolidinyl) 4-pyrimidinyl] 1-pipérazinyl] 1-propynyl] 17béta-hydroxy estra-4,9-dièn-3-one,
- 11béta-[4-(diméthylamino) phényl] 17alpha-[3-[4-[5,6-bis(diéthylamino) 2-pyridyl] 1-pipérazinyl] 1-propynyl] 17béta-hydroxy estra-4,9-dièn-3-one ou
- 11béta-[4-(diméthylamino) phényl] 17alpha-[3-[4-[3,6-bis(diéthylamino) 2-pyridyl] 1-pipérazinyl] 1-propynyl] 17béta-hydroxy estra-4,9-dièn-3-one ou
- 11béta-[4-(diméthylamino) phényl] 17alpha-[3-[4-[2,6-bis(1-pyrrolidinyl) 4-pyrimidinyl] 1-pipérazinyl] 1-propényl] 17béta-hydroxy estra-4,9-dièn-3-one ou
- 11béta-[4-(diméthylamino) phényl] 17alpha-[3-[4-[2,6-bis(1-pyrrolidinyl) 4-pyrimidinyl] 1-pipérazinyl] 1-propyl] 17béta-hydroxy estra-4,9-dièn-3-one

et leurs sels.

**13.** Procédé de préparation des composés de formule (I) telle que définie à la revendication 1, caractérisé en ce que l'on soumet, dans un solvant neutre et en présence d'une base, un composé de formule (II) choisi parmi

**soit a)** un composé de formule (IIa) :

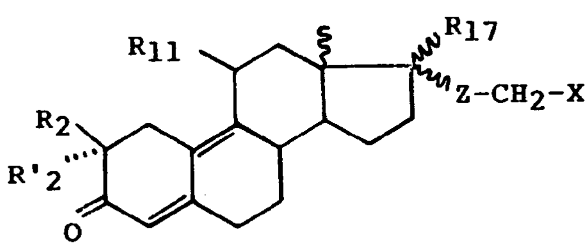

(IIa)

**soit b)** un composé de formule (IIb) :

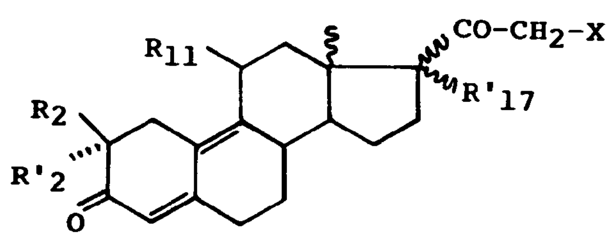

(IIb)

formules dans lesquelles X est un atome d'halogène et $R_2$, $R'_2$, $R_{11}$, les traits ondulés, $R_{17}$, $R'_{17}$ et Z ont les mêmes significations qu'à la revendication 1, à l'action d'un composé de formule (III) :

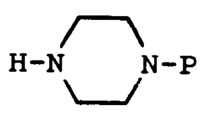

(III)

dans laquelle P a la même signification qu'à la revendication 1, et, le cas échéant, lorsque Z est un radical alcénylène ou alcynylène, l'on soumet les produits de formule (I) à une réaction d'hydrogénation partielle

ou totale et, si désiré, l'on soumet les produits de formule (I) à l'action d'un acide pour obtenir le sel correspondant.

**14.** Variante du procédé, selon la revendication 13, de préparation des composés de formule (I) dans laquelle $R_2$ et $R'_2$ représentent chacun un atome d'hydrogène, $R_{17}$ est un radical hydroxyle et $R'_{17}$ est un radical

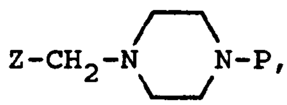

caractérisé en ce que l'on soumet un composé de formule (II') :

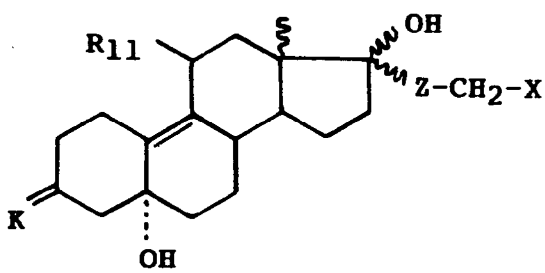

(II')

dans laquelle X est un atome d'halogène, $R_{11}$, les traits ondulés et Z ont la même signification qu'à la revendication 1 et K représente un groupement protecteur de la fonction cétone en 3, à l'action d'un composé de formule (III) :

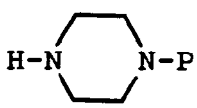

(III)

dans laquelle P a la même signification qu'à la revendication 1, puis soumet le produit de formule (XV) obtenu :

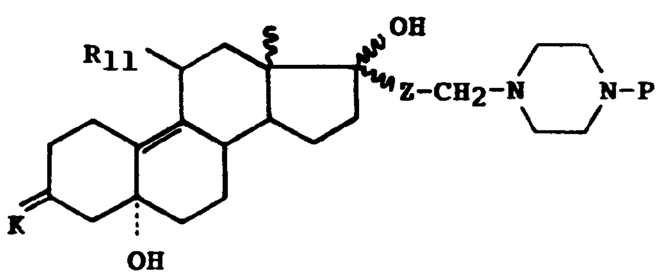

(XV)

à une réaction de deshydratation et d'hydrolyse susceptibles de libérer la fonction 3-céto delta-4, cette réaction étant précédée ou suivie d'une hydrogénation partielle ou totale éventuelle, lorsque Z est un radical alcynylène ou alcénylène, et si désiré, soumet les produits de formule (I) à l'action d'un acide pour obtenir le sel correspondant.

**15.** A titre de médicament, les composés de formule (I) telle que définie à l'une quelconque des revendications 1 à 11 et leurs sels avec les acides pharmaceutiquement acceptables.

**16.** A titre de médicament, selon la revendication 15, l'un quelconque des composés définis à la revendication 12 et leurs sels pharmaceutiquement acceptables.

**17.** Les compositions pharmaceutiques renfermant comme principe actif au moins l'un quelconque des médicaments tel que défini à la revendication 15 ou 16.

**18.** A titre de produits industriels nouveaux, les composés de formule (II), (II') ou (XV).

**19.** Les produits industriels nouveaux, selon la revendication 18, répondant à la formule :
- 17alpha-(3-bromo 1-propynyl) 11béta-[4-(diméthylamino) phényl] 17béta-hydroxy estra-4,9-dièn-3-one et
- 3,3-(éthanediyl bis oxy) 17alpha-(3-bromo 1-propynyl) 11béta-[4-(diméthylamino) phényl] 5alpha,17béta-dihydroxy estra-9-ène.

## Patentansprüche

**1.** Verbindungen der Formel (I)

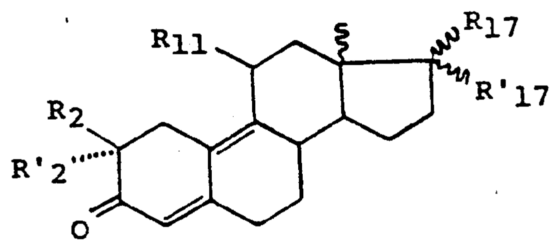

(I)

in der
- $R_2$ und $R'_2$, gleich oder verschieden, ein Wasserstoffatom oder einen Methylrest darstellen,
- $R_{11}$ einen organischen Rest mit 1 bis 18 Kohlenstoffatomen bedeutet, der gegebenenfalls ein oder mehrere Heteroatome umfaßt, wobei das unmittelbar an den Kohlenstoff in Stellung 11 angrenzende Atom ein Kohlenstoffatom ist.
- die Wellenlinien in Stellung 13 und 17 anzeigen, daß in dem Fall. wo der Methylrest in Stellung 13 in $\alpha$ ist, der Substituent $R_{17}$ in $\alpha$-Konfiguration und der Substituent $R'_{17}$ in $\beta$-Konfiguration vorliegen. und wenn der Methylrest in Stellung 13 in $\beta$ ist, der Substituent $R_{17}$ in $\beta$-Konfiguration und der Substituent $R'_{17}$ in $\alpha$-Konfiguration vorliegen,

und worin $R_{17}$ und $R'_{17}$ bedeuten:

a) <u>entweder</u> ist $R_{17}$ ein Hydroxylrest oder ein Acyloxyrest, abgeleitet von einer organischen Carbonsäure mit 1 bis 18 Kohlenstoffatomen, und $R'_{17}$ ist ein Rest

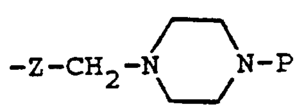

worin Z eine Einfachbindung, einen Alkylenrest mit 1 bis 5 Kohlenstoffatomen. einen Alkenylenrest oder einen Alkinylenrest mit 2 bis 5 Kohlenstoffatomen darstellt, und P einen Pyrimidinylrest oder einen Pyridylrest bedeutet, substituiert durch ein oder zwei gleiche oder verschiedene Reste, ausgewählt unter den Resten Amino, Alkylamino, Dialkylamino oder den Amino-Heterocyclen mit 5 oder 6 Ringgliedern. gegebenenfalls substituiert durch einen Alkylrest mit 1 bis 3 Kohlenstoffatomen.

b) <u>oder</u> $R_{17}$ ist ein Rest

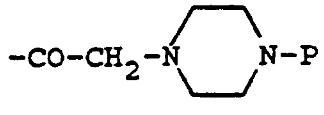

worin P die gleiche Bedeutung wie vorstehend besitzt und $R'_{17}$ ein Wasserstoffatom, ein Hydroxylrest oder ein Acyloxyrest ist,
und die Salze dieser Verbindungen.

**2.** Verbindungen der Formel (I) wie in Anspruch 1 definiert, in der P einen Pyrimidinylrest oder einen Pyridylrest darstellt, substituiert durch zwei gleiche oder verschiedene Reste, ausgewählt unter den Dialkylaminoresten oder den Amino-Heterocyclen mit 5 oder 6 Ringgliedern.

**3.** Verbindungen der Formel (I) wie in Anspruch 1 oder 2 definiert, in der P darstellt:
- einen 2,6-bis-(1-Pyrrolidinyl)-4-pyrimidinyl-Rest,
- einen 5,6-bis-(Diethylamino)-2-pyridyl-Rest oder
- einen 3,6-bis-(Diethylamino)-2-pyridyl-Rest.

**4.** Verbindungen der Formel (I) wie in irgendeinem der Ansprüche 1 bis 3 definiert, worin der Methylrest in Stellung 13 in β-Konfiguration vorliegt.

**5.** Verbindungen der Formel (I) wie in irgendeinem der Ansprüche 1 bis 4 definiert, in der $R_{11}$ einen Arylrest oder einen Aralkylrest darstellt, der eine Aminfunktion

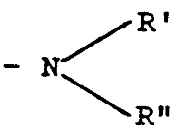

trägt, worin R' und R" einen Alkylrest mit 1 bis 8 Kohlenstoffatomen oder einen primären, sekundären oder tertiären Alkylrest mit 1 bis 8 Kohlenstoffatomen darstellen. umfassend ein oder mehrere Heteroatome, ausgewählt aus der durch Sauerstoff, Stickstoff und Schwefel gebildeten Gruppe, von denen mindestens eines ein Stickstoffatom ist, oder substituiert durch einen Heterocyclus mit mindestens einem Stickstoffatom.

**6.** Verbindungen der Formel (I) wie in irgendeinem der Ansprüche 1 bis 4 definiert, in der $R_{11}$ darstellt:
einen 2,3- oder 4-Pyridylrest,
einen Rest

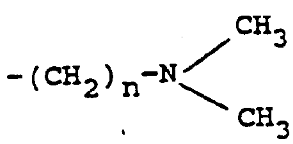

($n \geqq 3$),
einen Rest:

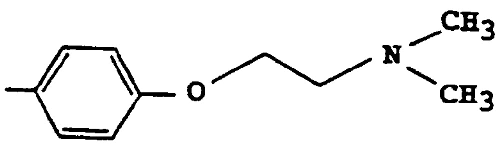

,

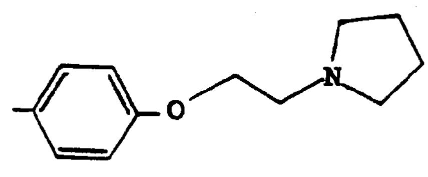

,

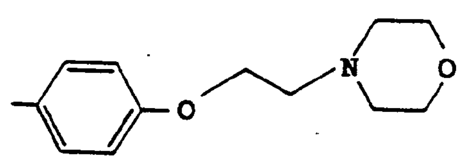

,

einen Rest:

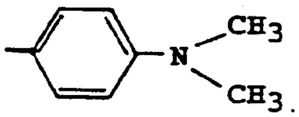 , 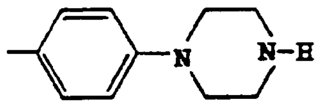 , 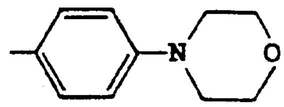

einen Rest:

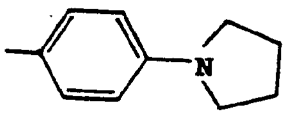 , 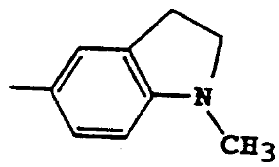

oder einen Rest:

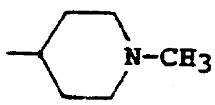

**7.** Verbindungen der Formel (I) wie in irgendeinem der Ansprüche 1 bis 4 definiert, in der $R_{11}$ einen Rest Thienyl, Furyl, Cycloalkyl mit 3 bis 6 Kohlenstoffatomen oder Phenyl darstellt, gegebenenfalls substituiert durch einen oder mehrere Reste, ausgewählt unter den Resten Hydroxy, Halogen, Trifluormethyl, Alkyl, Alkyloxy, Alkylthio, gegebenenfalls oxidiert in Form von Sulfoxid oder Sulfon.

**8.** Verbindungen der Formel (I) wie in irgendeinem der Ansprüche 1 bis 4 und 7 definiert, in der $R_{11}$ einen Phenylrest darstellt, substituiert durch einen Rest, ausgewählt aus der durch die folgenden Reste gebildeten Gruppe: Chlor, Fluor, Methylthio, Methylsulfonyl, Methoxy, Hydroxy, Allyloxy und Acyl, wie Acetyl.

**9.** Verbindungen der Formel (I) wie in irgendeinem der Ansprüche 1 bis 8 definiert, in der $R_2$ und $R'_2$, untereinander gleich, jeweils ein Wasserstoffatom darstellen.

**10.** Verbindungen der Formel (I) wie in irgendeinem der Ansprüche 1 bis 9 definiert, in der $R_{17}$ ein Hydroxylrest ist, wenn $R'_{17}$ einen Rest

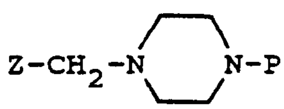

darstellt.

**11.** Verbindungen der Formel (I) wie in irgendeinem der Ansprüche 1 bis 10 definiert, in der Z gleich -C≡C- oder -CH=CH- oder $-CH_2-CH_2-$ ist, wenn $R'_{17}$ einen Rest

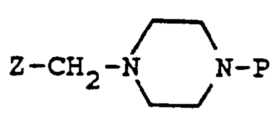

bedeutet.

**12.** Verbindungen, die irgendeiner der folgenden Formeln entsprechen:

- 11β-[4-(Dimethylamino)-phenyl]-17α-[3-[4-[2,6-bis-(1-pyrrolidinyl)-4-pyrimidinyl]-1-piperazinyl]-1-propinyl]-17β-hydroxy-estra-4,9-dien-3-on,
- 11β-[4-(Dimethylamino)-phenyl]-17α-[3-[4-[5,6-bis-(diethylamino)-2-pyridyl]-1-piperazinyl]-1-propinyl]-17β-hydroxy-estra-4,9-dien-3-on, oder
- 11β-[4-(Dimethylamino)-phenyl]-17α-[3-[4-[3,6-bis-(diethylamino)-2-pyridyl]-1-piperazinyl]-1-propinyl]-17β-hydroxy-estra-4,9-dien-3-on, oder
- 11β-[4-(Dimethylamino)-phenyl]-17α-[3-[4-[2,6-bis-(1-pyrrolidinyl)-4-pyrimidinyl]-1-piperazinyl]-1-propenyl]-17β-hydroxy-estra-4,9-dien-3-on, oder
- 11β-[4-(Dimethylamino)-phenyl]-17α-[3-[4-[2,6-bis-(1-pyrrolidinyl)-4-pyrimidinyl]-1-piperazinyl]-1-propyl]-17β-hydroxy-estra-4,9-dien-3-on, und ihre Salze.

**13.** Verfahren zur Herstellung der Verbindungen der Formel (I) wie in Anspruch 1 definiert, dadurch gekennzeichnet, daß man in einem neutralen Lösungsmittel und in Anwesenheit einer Base eine Verbindung der Formel (II), ausgewählt unter entweder a) einer Verbindung der Formel (IIa)

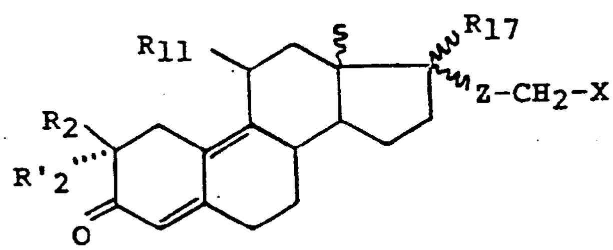

(IIa)

oder b) einer Verbindung der Formel (IIb)

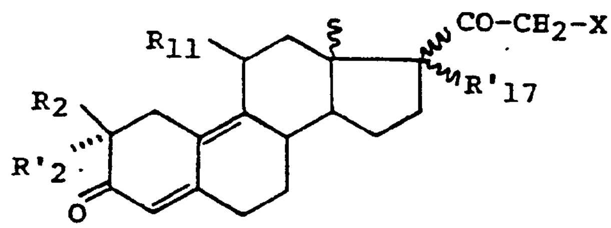

(IIb)

in denen X ein Halogenatom ist und $R_2$, $R'_2$, $R_{11}$, die Wellenlinien, $R_{17}$, $R'_{17}$ und Z die gleichen Bedeutungen wie in Anspruch 1 besitzen, der Einwirkung einer Verbindung der Formel (III)

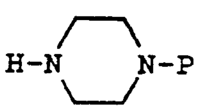

(III)

unterzieht, in der P die gleiche Bedeutung wie in Anspruch 1 besitzt, und man gegebenenfalls, wenn Z ein Alkenylenrest oder Alkinylenrest ist, die Produkte der Formel (I) einer Reaktion zur teilweisen oder vollständigen Hydrierung unterwirft, und man, wenn gewünscht, die Produkte der Formel (I) der Einwirkung einer Säure unterzieht, um das entsprechende Salz zu erhalten.

**14.** Variante des Verfahrens nach Anspruch 13 zur Herstellung der Verbindungen der Formel (I), in der $R_2$ und $R'_2$ jeweils ein Wasserstoffatom darstellen, $R_{17}$ ein Hydroxylrest ist und $R'_{17}$ einen Rest

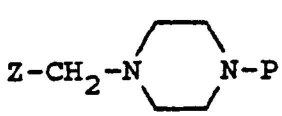

bedeutet, dadurch gekennzeichnet, daß man eine Verbindung der Formel (II')

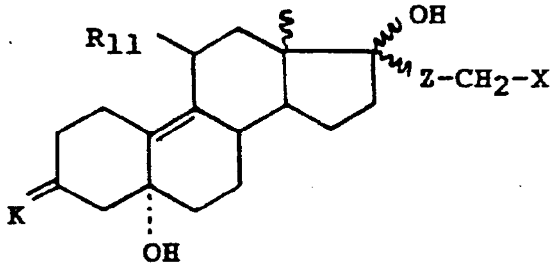

(II')

in der X ein Halogenatom ist, $R_{11}$, die Wellenlinien und Z die gleichen Bedeutungen wie in Anspruch 1 besitzen und K eine Schutzgruppe für die Ketonfunktion in Stellung 3 bedeutet, der Einwirkung einer Verbindung der Formel (III)

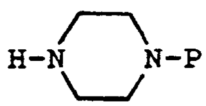

(III)

unterzieht, in der P die gleiche Bedeutung wie in Anspruch 1 besitzt, und man anschließend das erhaltene Produkt der Formel (XV)

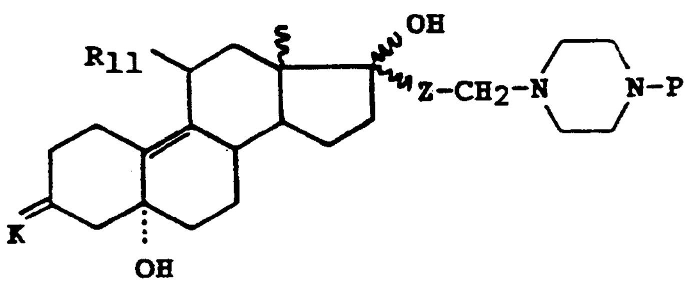

(XV)

einer Reaktion zur Dehydratation und Hydrolyse unterzieht, die geeignet ist, die 3-Keto-δ-4-Funktion freizusetzen, wobei diese Reaktion vor oder nach einer eventuellen teilweisen oder vollständigen Hydrierung erfolgt, wenn Z ein Alkenylenrest oder Alkinylenrest ist, und man, wenn gewünscht, die Produkte der Formel (I) der Einwirkung einer Säure unterzieht, um das entsprechende Salz zu erhalten.

**15.** Als Medikament die Verbindungen der Formel (I) wie in irgendeinem der Ansprüche 1 bis 11 definiert und ihre Salze mit pharmazeutisch akzeptablen Säuren.

**16.** Als Medikament nach Anspruch 15 irgendeine der in Anspruch 12 definierten Verbindungen und ihre pharmazeutisch akzeptablen Salze.

**17.** Pharmazeutische Zusammensetzungen, umfassend als Wirkstoff mindestens eines der Medikamente wie in Anspruch 15 oder 16 definiert.

**18.** Als neue industrielle Produkte die Verbindungen der Formel (II), (II') oder (XV).

**19.** Als neue industrielle Produkte gemäß Anspruch 18 die Verbindungen, die der Formel entsprechen:
- 17α-(3-Brom-1-propinyl)-11β-[4-(dimethylamino)-phenyl]-17β-hydroxy-estra-4,9-dien-3-on und
- 3,3-(Ethandiyl-bis-oxy)-17α-(3-Brom-1-propinyl)-11β-[4-(dimethylamino)- phenyl]-5α,17β-dihydroxy-estra-9-en.

## Claims

1. The compounds of formula (I):

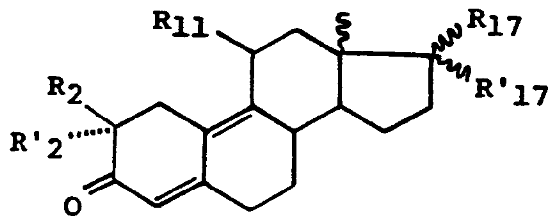

(I)

in which:
- $R_2$ and $R'_2$, identical or different, represent a hydrogen atom or a methyl radical,
- $R_{11}$ represents an organic radical containing 1 to 18 carbon atoms and optionally one or more heteroatoms, the atom immediately adjacent to the carbon atom in position 11 being a carbon atom,
- the wavy lines in position 13 and 17 indicate that when the methyl radical in position 13 is in alpha configuration, the $R_{17}$ substituent is in alpha configuration and the $R'_{17}$ substituent is in beta configuration and that when the methyl radical in position 13 is in beta configuration, the $R_{17}$ substituent is in beta configuration and the $R'_{17}$ substituent is in alpha configuration and in which $R_{17}$ and $R'_{17}$ are such that

**either a)** $R_{17}$ is a hydroxyl or an acyloxy radical derived from a carboxylic organic acid containing 1 to 18 carbon atoms and $R'_{17}$ is a

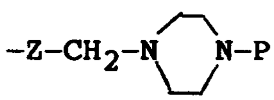

radical in which Z is a single bond, an alkylene radical containing 1 to 5 carbon atoms, an alkenylene radical or an alkynylene radical containing 2 to 5 carbon atoms and P represents a pyrimidinyl radical or a pyridyl radical, substituted by one or two identical or different radicals chosen from the following radicals: amino, alkylamino, dialkylamino or amino heterocycles with 5 or 6 members, optionally substituted by an alkyl radical having 1 to 3 carbon atoms,
**or b)** $R_{17}$ is a

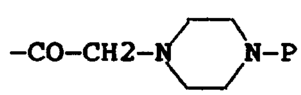

radical in which P has the same meaning as above and $R'_{17}$ is a hydrogen atom, a hydroxyl or an acyloxy radical,
and the salts of these compounds.

2. The compounds of formula (I) as defined in claim 1 in which P represents a pyrimidinyl radical or a pyridyl radical substituted by two identical or different radicals chosen from dialkylamino radicals or amino heterocycles with 5 or 6 members.

3. The compounds of formula (I) as defined in claim 1 or 2 in which P represents
   - a 2,6-bis(1-pyrrolidinyl) 4-pyrimidinyl radical,
   - a 2,6-bis(diethylamino) 2-pyridyl radical or
   - a 3,6-bis(diethylamino) 2-pyridyl radical.

4. The compounds of formula (I) as defined in any one of claims 1 to 3 in which the methyl radical in position 13 is in beta configuration.

5. The compounds of formula (I) as defined in any one of claims 1 to 4 in which $R_{11}$ represents an aryl or

aralkyl radical carrying an amine function:

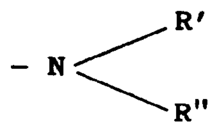

in which R' and R" represent an alkyl radical containing 1 to 8 carbon atoms or a primary, secondary or tertiary alkyl radical containing 1 to 8 carbon atoms, containing one or more heteroatoms chosen from the group constituted by oxygen, nitrogen and sulphur, of which at least one is a nitrogen atom, or substituted by a heterocycle containing at least one nitrogen atom.

**6.** The compounds of formula (I) as defined in any one of claims 1 to 4 in which $R_{11}$ represents a 2,3 or 4-pyridyl radical,
a

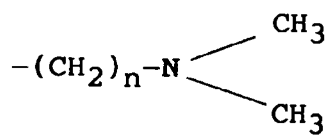

radical
($n \geqq 3$),
a radical:

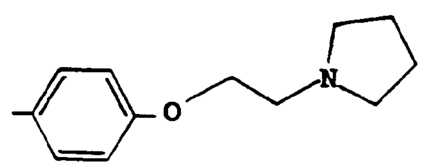

,

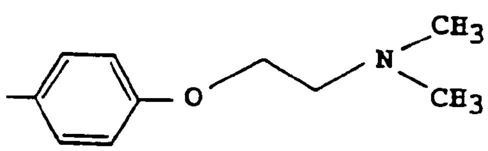

,

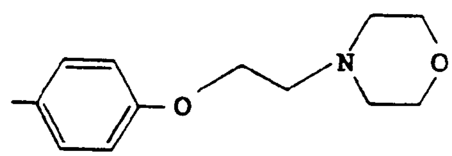

,

a radical:

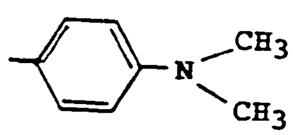

,

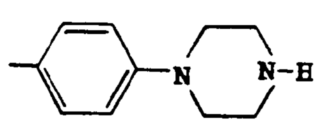

,

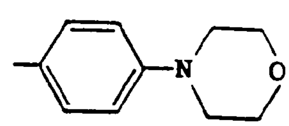

a radical:

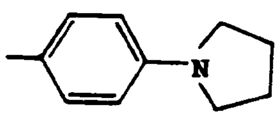

,

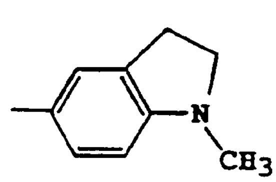

or a radical:

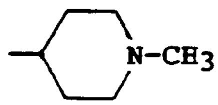

7. The compounds of formula (I) as defined in any one of claims 1 to 4 in which $R_{11}$ represents one of the following radicals: thienyl, furyl, cycloalkyl having 3 to 6 carbon atoms or phenyl optionally substituted by one or more radicals chosen from the following radicals: hydroxy, halogen, trifluoromethyl, alkyl, alkyloxy, alkylthio optionally oxidized in the form of the sulphoxide or sulphone.

8. The compounds of formula (I) as defined in claims 1 to 4 and 7 in which $R_{11}$ represents a phenyl radical substituted by a radical chosen from the group formed by the following radicals: chloro, fluoro, methylthio, methylsulphonyl, methoxy, hydroxy, allyloxy and acyl such as acetyl.

9. The compounds of formula (I) as defined in any one of claims 1 to 8 in which $R_2$ and $R'_2$, being identical, each represent a hydrogen atom.

10. The compounds of formula (I) as defined in any one of claims 1 to 9 in which $R_{17}$ is a hydroxyl radical when $R'_{17}$ represents a

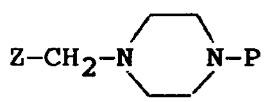

radical.

11. The compounds of formula (I) as defined in any one of claims 1 to 10 in which Z = -C=C- or Z = -CH=CH- or Z = $-CH_2-CH_2-$ when $R'_{17}$ represents a

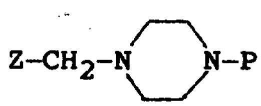

radical.

12. The compounds corresponding to any one of the following formulae:
   - 11beta-[4-(dimethylamino) phenyl] 17alpha-[3-[4-[(2,6-bis (1-pyrrolidinyl) 4-pyrimidinyl] 1-piperazinyl] 1-propynyl] 17beta-hydroxy estra-4,9-dien-3-one,
   - 11beta-[4-(dimethylamino) phenyl] 17alpha-[3-[4-[5,6-bis (diethylamino) 2-pyridyl] 1-piperazinyl] 1-propynyl] 17beta-hydroxy estra-4,9-dien-3-one or
   - 11beta-[4-(dimethylamino) phenyl] 17alpha-[3-[4-[3,6-bis (diethylamino) 2-pyridyl] 1-piperazinyl] 1-propynyl] 17beta-hydroxy estra-4,9-dien-3-one or
   - 11beta-[4-(dimethylamino) phenyl] 17alpha-[3-[4-[2,6-bis (1-pyrrolidinyl) 4-pyrimidinyl] 1-piperazinyl] 1-propenyl] 17beta-hydroxy estra-4,9-dien-3-one or
   - 11beta-[4-(dimethylamino) phenyl] 17alpha-[3-[4-[2,6-bis (1-pyrrolidinyl) 4-pyrimidinyl] 1-piperazinyl] 1-propyl] 17beta-hydroxy estra-4,9-dien-3-one

   and their salts.

13. Preparation process for the compounds of formula (I) as defined in claim 1, characterized in that a compound of formula (II) chosen from
**either a)** a compound of formula (IIa):

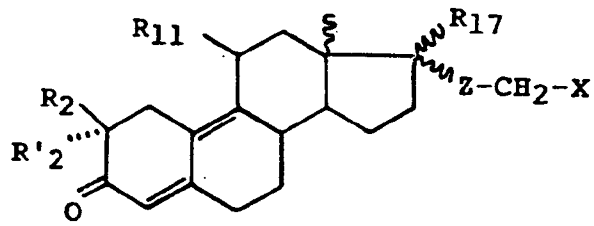

(IIa)

**or b)** a compound of formula (IIb):

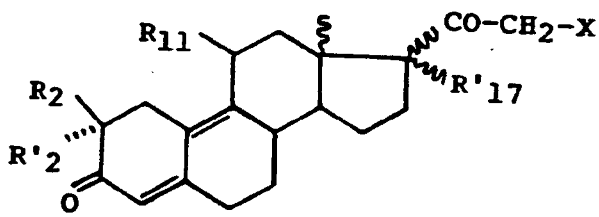

(IIb)

in which formulae X is a halogen atom and $R_2$, $R'_2$, $R_{11}$, the wavy lines, $R_{17}$, $R'_{17}$ and Z have the same meanings as in claim 1, is subjected, in a neutral solvent and in the presence of a base, to the action of a compound of formula (III):

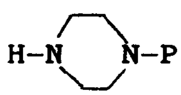

(III)

in which P has the same meaning as in claim 1, and, if appropriate, when Z is an alkenylene or alkynylene radical, the products of formula (I) are subjected to a partial or total hydrogenation reaction and, if desired, the products of formula (I) are subjected to the action of an acid in order to obtain the corresponding salt.

**14.** Variant of the process, according to claim 13, for the preparation of the compounds of formula (I) in which $R_2$ and $R'_2$ each represent a hydrogen atom, $R_{17}$ is a hydroxyl radical and $R'_{17}$ is a

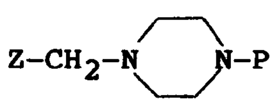

radical, characterized in that a compound of formula (II'):

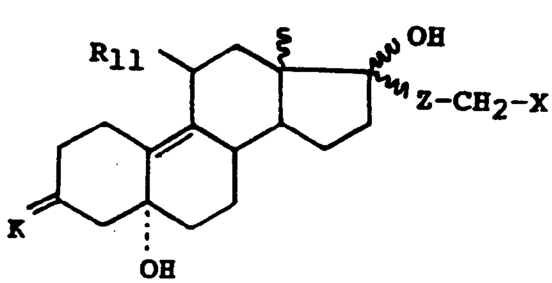

(II')

in which X is a halogen atom, $R_{11}$, the wavy lines and Z have the same meaning as in claim 1 and K represents a protector group of the ketone function in position 3, is subjected to the action of a compound of formula (III):

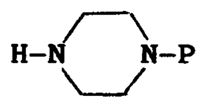

(III)

in which P has the same meaning as in claim 1, then the product of formula (XV) obtained:

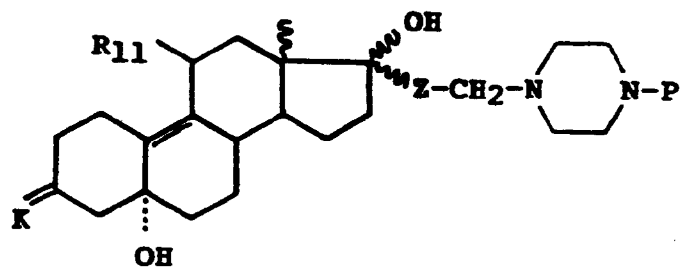

(XV)

is subjected to a dehydration and hydrolysis reaction capable of releasing the 3-keto delta-4 function, this reaction being preceded or followed by an optional partial or total hydrogenation, when Z is an alkynylene or alkenylene radical, and if desired, the products of formula (I) are subjected to the action of an acid in order to obtain the corresponding salt.

**15.** As a medicament, the compounds of formula (I) as defined in any one of claims 1 to 11 and their salts with pharmaceutically acceptable acids.

**16.** As a medicament according to claim 15, any one of the compounds defined in claim 12 and their pharmaceutically acceptable salts.

**17.** The pharmaceutical compositions containing at least any one of the medicaments as defined in claim 15 or 16 as active ingredient.

**18.** As new industrial products, the compounds of formulae (II), (II') or (XV).

**19.** The new industrial products, according to claim 18, corresponding to the formula:
- 17alpha-(3-bromo 1-propynyl) 11beta-[4-(dimethylamino) phenyl] 17beta-hydroxy estra-4,9-dien-3-one and
- 3,3-(ethanediyl bis oxy) 17alpha-(3-bromo 1-propynyl) 11beta-[4-(dimethylamino) phenyl] 5alpha,17beta-dihydroxy estra-9-ene.